# EUROPEAN PATENT SPECIFICATION

(11) **EP 3 717 024 B1**
(45) Date of publication and mention of the grant of the patent: **17.09.2025**
(21) Application number: 18883916.1
(22) Date of filing: 28.11.2018
(51) Int. Cl.: A61K 48/00, A61K 9/00, A61K 31/7088, A61P 27/02

(54) **GENE THERAPY FOR OCULAR IMPROVEMENT**
GENTHERAPIE ZUR AUGENVERBESSERUNG
THÉRAPIE GÉNIQUE POUR L'AMÉLIORATION DES FONCTIONS OCULAIRES

(30) Priority: 29.11.2017 US 201762592033 P; 22.10.2018 US 201862748788 P
(43) Date of publication of application: 07.10.2020
(73) Proprietor: Copernicus Therapeutics, Inc., Cleveland OH 44106 (US); Clearside Biomedical, Alpharetta, GA 30005 (US)
(72) Inventor: TARABORELLI, Donna, Alpharetta Georgia 30005 (US); YOO, Jesse, Alpharetta, Georgia 30005 (US); NORONHA, Glenn, Alpharetta, Georgia 30005 (US); COOPER, Mark J., Cleveland, Ohio 44106 (US); MOEN, Robert C., Cleveland, Ohio 44106 (US); WHITE, Daniel, Alpharetta, Georgia 30005 (US)
(74) Representative: Tombling, Adrian George
(86) International application number: PCT/US2018/062712
(87) International publication number: WO 2019/108570

(56) References cited:
- WO-A1-2008/137066
- WO-A1-2011/017313
- WO-A1-2014/074823
- US-A1- 2016 243 257
- US-A1- 2016 310 417
- US-B2- 8 017 577
- CHERYL L. ROWE-RENDLEMAN ET AL: "Drug and Gene Delivery to the Back of the Eye: From Bench to Bedside", INVESTIGATIVE OPTHALMOLOGY & VISUAL SCIENCE, vol. 55, no. 4, 28 April 2014 (2014-04-28), US, pages 2714 - 2730, XP055516116, ISSN: 1552-5783, DOI: 10.1167/iovs.13-13707
- ADIJANTO, J. ET AL.: "Nanoparticle-based technologies for retinal gene therap y", EUROPEAN JOURNAL OF PHARMACEUTICS AND BIOPHARMACEUTICS, vol. 95, 12 January 2015 (2015-01-12), pages 353 - 367, XP029296799, doi:10.1016/j.ejpb.2014.12.028
- FARJO, R. ET AL.: "Efficient non-viral ocular gene transfer with compacted DNA nanoparticles", PLOS ONE, vol. 1, December 2006 (2006-12-01), pages 1 - 8, XP055395261

## Description

### TECHNICAL FIELD OF THE INVENTION

This invention is related to the area of gene therapy. In particular, it relates to gene therapy to the eye.

### BACKGROUND OF THE INVENTION

Effective and long lasting delivery and expression of nucleic acids to eyes remain issues that have hindered widespread use of gene therapy for treating ocular diseases. There is a continuing need in the art to develop methods for effective delivery and long lasting expression of nucleic acids without causing appreciable damage to the eye.

### SUMMARY OF THE INVENTION

The invention is as defined in the appended claims. The invention enables the treatment of ocular disorders without causing appreciable damage to the eye.

### BRIEF DESCRIPTION OF THE DRAWINGS

Fig. 1 shows analysis of the choroids of rabbit eyes after suprachoroidal (SC) or subretinal (SR) injection of nanoparticles of a luciferase gene. The nanoparticles were in either the rod or ellipsoid shape, depending on the counterion used at the time of making the nanoparticles. A negative control group received SC dosing of saline. OS (oculus sinister) represents the injected left eye and OD (oculus dexter) the control right eye.
Fig. 2 shows analysis of the retinae of rabbit eyes after suprachoroidal (SC) or subretinal (SR) injection of nanoparticles of a luciferase gene. The nanoparticles were in either the rod or ellipsoid shape, depending on the counterion used at the time of making the nanoparticles. A negative control group received SC dosing of saline. OS (oculus sinister) represents the injected left eye and OD (oculus dexter) the control right eye.
Fig. 3 shows autosomal recessive retinitis pigmentosa (RP) mutations.
Fig. 4 shows autosomal dominant retinitis pigmentosa (RP) mutations.
Fig. 5 shows X-linked retinitis pigmentosa (RP) mutations.

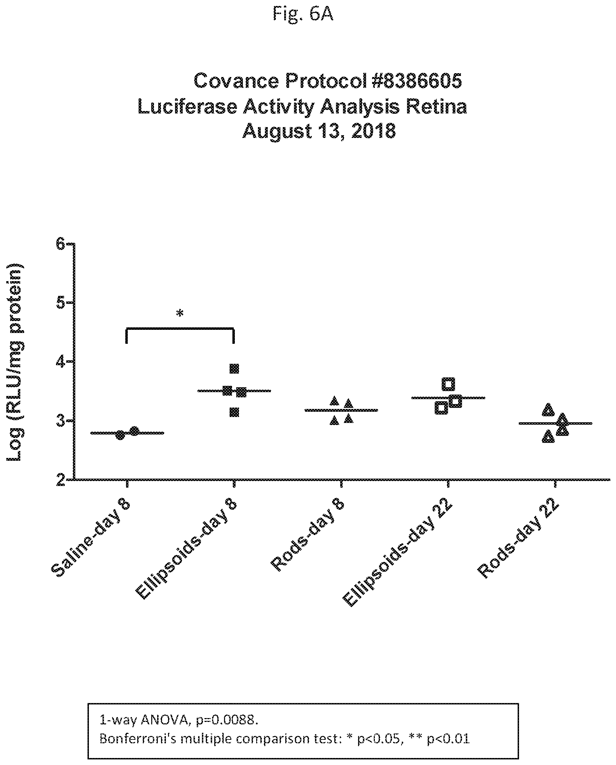

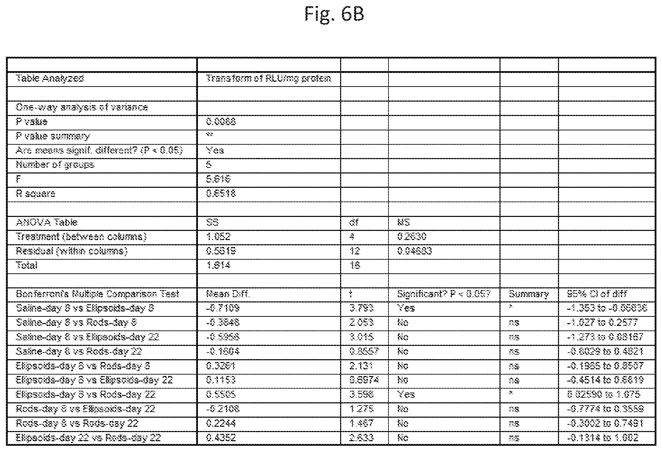

Figs. 6A-6B show luciferase activity analysis of the monkey retina and the statistical analysis of the data, respectively.

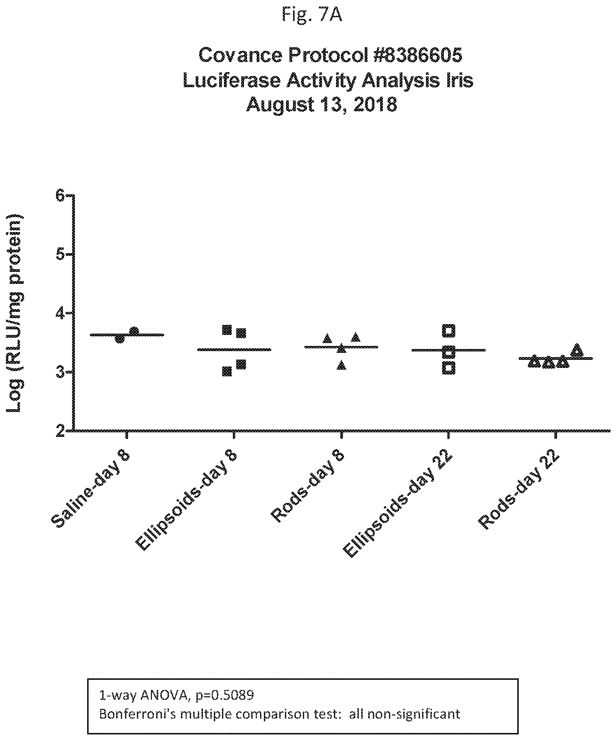

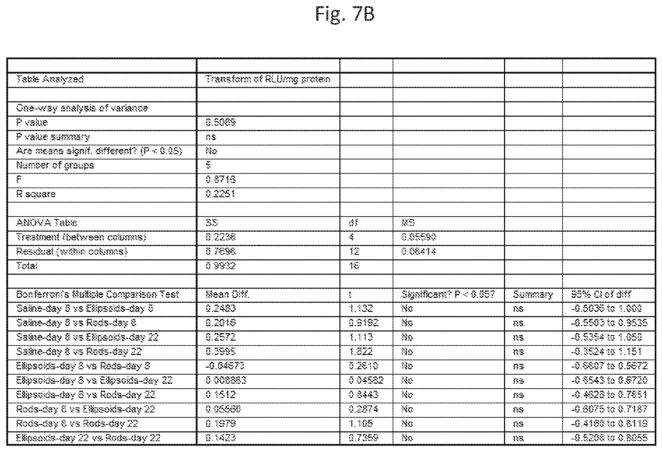

Figs. 7A-7B show luciferase activity analysis of the monkey iris and the statistical analysis of the data, respectively.

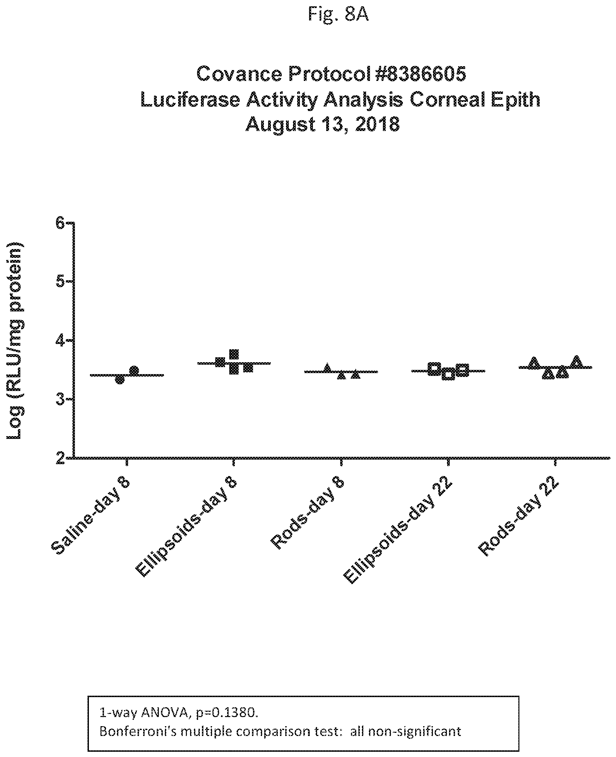

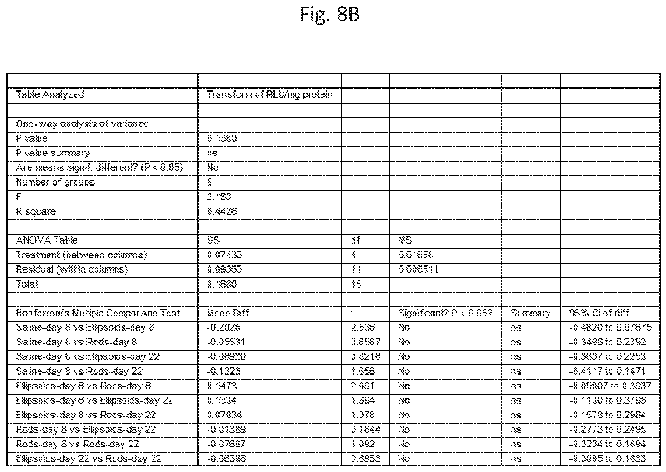

Figs. 8A-8B show luciferase activity analysis of the monkey corneal epithelium and the statistical analysis of the data, respectively.

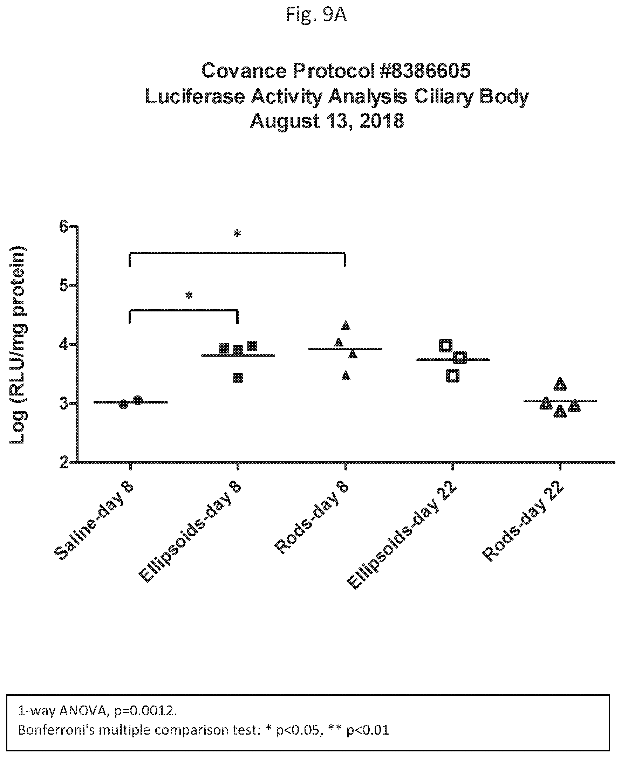

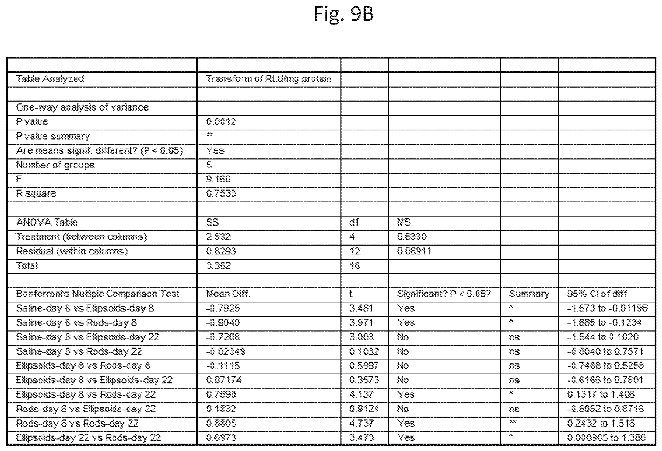

Figs. 9A-9B show luciferase activity analysis of the monkey ciliary body and the statistical analysis of the data, respectively.

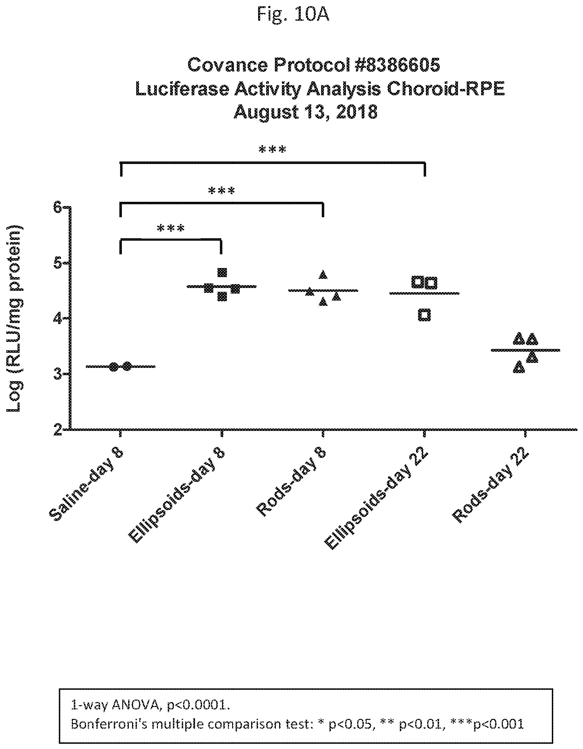

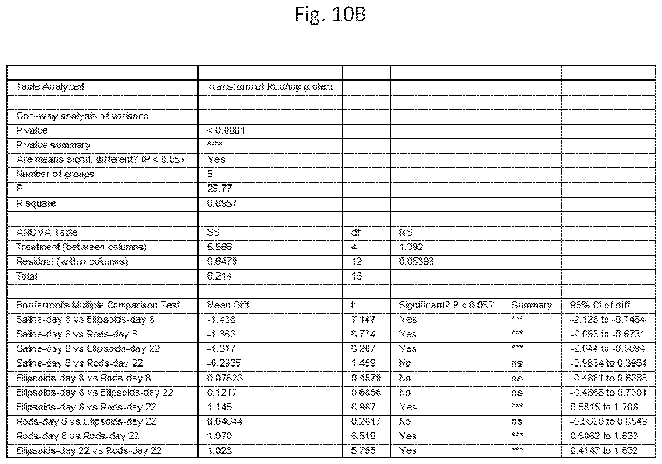

Figs. 10A-10B show luciferase activity analysis of the choroid-retinal pigment epithelium (RPE) and the statistical analysis of the data, respectively.

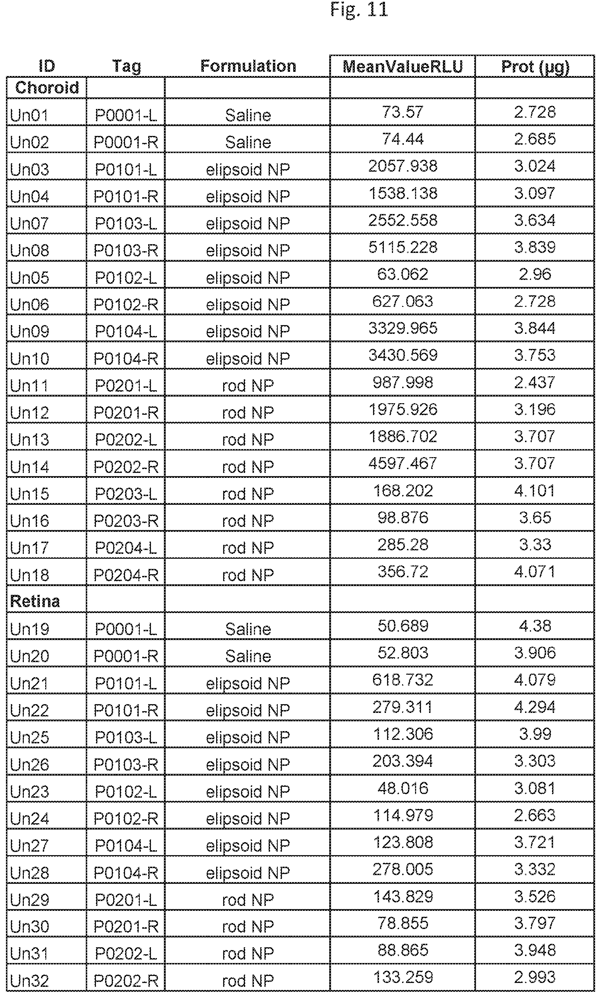

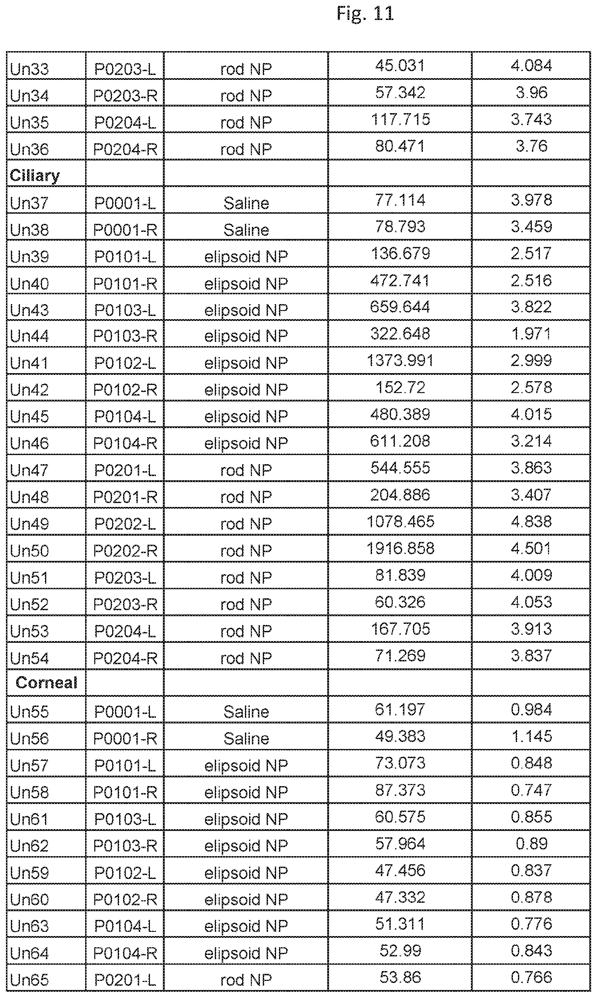

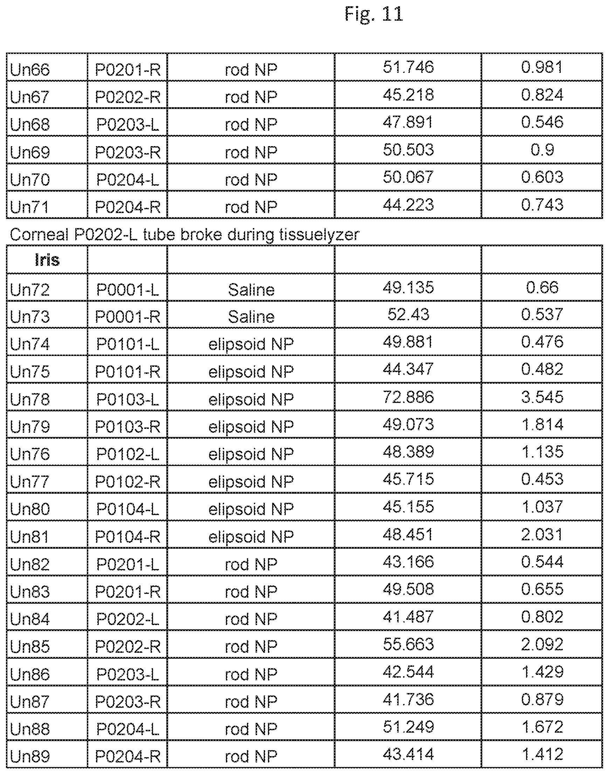

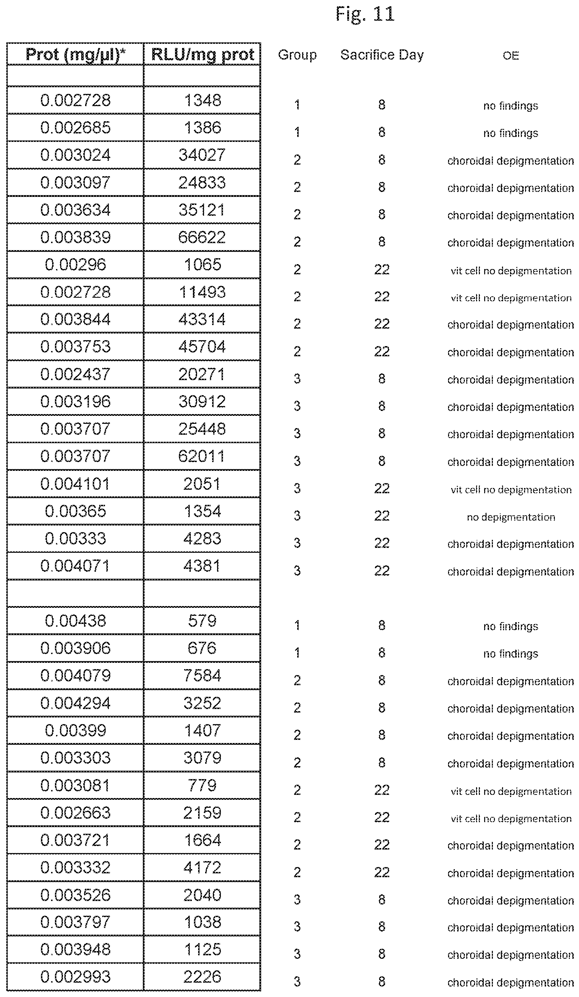

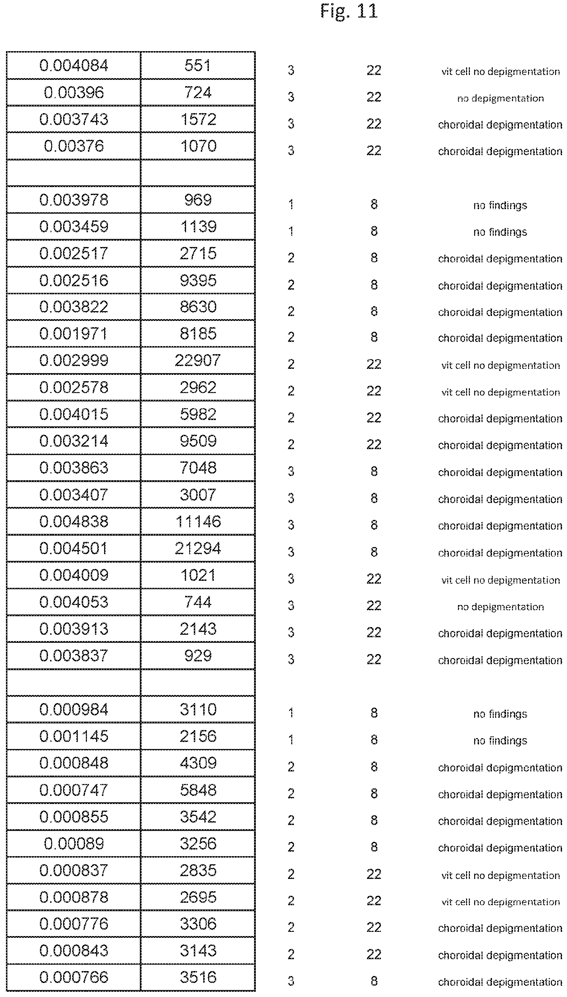

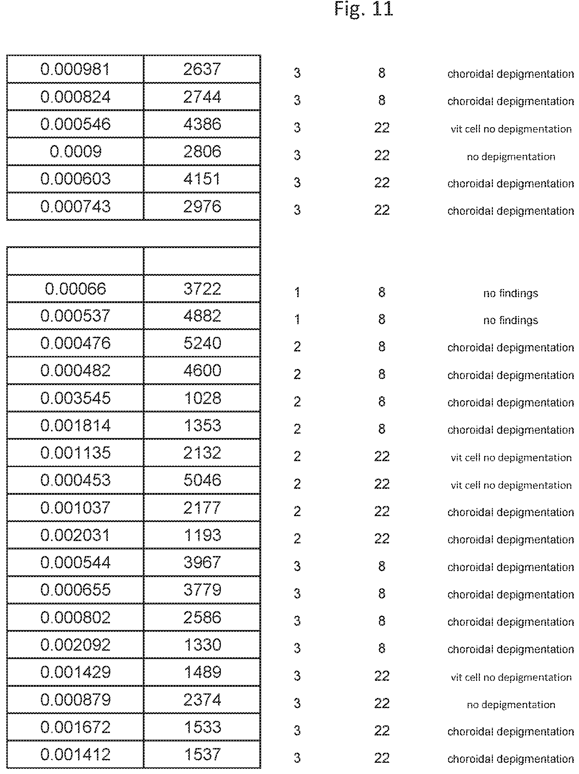

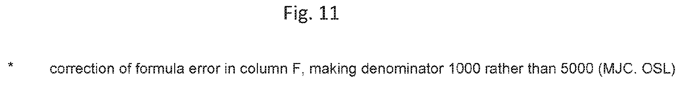

Fig. 11 provides raw data for each animal and tissue shown in Figs. 6A-10B.

### DETAILED DESCRIPTION OF THE INVENTION

References to methods of treatment herein are, with respect to the claimed invention, to be interpreted as references to formulations, compounds, pharmaceutical compositions and medicaments of the present invention for use in such methods.

The inventors have developed methods for treating eye disorders that involve suprachoroidal delivery of charge-neutral nanoparticles each comprising a single molecule of nucleic acid compacted to its smallest possible size. Briefly, a formulation is non-surgically administered to the (SCS) of an eye of a mammal. Typically the mammal has an ocular disorder. Desirably, the nanoparticles are delivered in an amount sufficient to elicit a therapeutic response to the ocular disorder.

Conditions under which the nanoparticles are made lead to nanoparticles of different shape. For example using an acetate counterion to the polycation used to condense the nucleic acid, such as polylysine, leads to rod-shaped nanoparticles. Using trifluoroacetate as a counterion to the polycation leads to ellipsoid-shaped nanoparticles. Ellipsoids typically have a minor diameter of less than 45 nm, less than 40 nm, less than 35 nm, less than 30 nm, less than 25 nm, or less than 20 nm, but greater than 15 nm. Rods typically have a diameter of between about 8-11, 7-12, or 6-13 nm. Other cations may be used to achieve shapes which may be advantageous or useful. See, *e.g.,* U.S. Patent 8,017,577. The polycation used for neutralizing the charge of the nucleic acid may be modified to achieve advantageous properties. For example, polylysine may be substituted with polyethylene glycol. This may increase the expression, delivery, or stability of the nanoparticles so made.

Nucleic acids which are made into nanoparticles are not as size limited as when using a viral vector. But it may be desirable that the nanoparticles themselves be sufficiently small so that they can efficiently access the nucleus. In some embodiments the nucleic acid is less than 30 kb or less than 30 kbp. In other the nucleic acid may be less than 25 kb or less than 25 kbp, less than 20 kb or less than 20 kbp, less than 15 kb or less than 15 kbp, less than 10 kb or less than 10 kbp, less than 5 kb or less than 5 kbp, or less than 1 kb or less than 1 kbp. Typically a nucleic acid will be at least 0.5 kbp or 0.5 kb, at least 1 kb or 1 kbp, at least 5 kb, or at least 5 kbp. The nucleic acid may be composed of RNA or DNA, may be double stranded or single stranded, or may comprise nucleic acid derivatives containing modified bases or backbone.

An ocular disorder can be treated according to the invention. Such include, without limitation uveitis, glaucoma, macular edema, diabetic macular edema, retinopathy, age-related macular degeneration, scleritis, optic nerve degeneration, geographic atrophy, choroidal disease, ocular sarcoidosis, optic neuritis, choroidal neovascularization, ocular cancer, retinitis pigmentosa, juvenile onset macular degeneration, a genetic disease, autoimmune diseases affecting the posterior segment of the eye, retinitis or corneal ulcers. These also include choroidal disorders without limitation, such as choroidal neovascularization, choroidal vascular proliferation, polypoidal choroidal vasculopathy, central sirrus choroidopathy, a multi-focal choroidopathy or choroidal dystrophy. The payload of the nanoparticle may differ for different disorders. The payload may encode, *e.g.,* a therapeutic protein, an inhibitory protein, or a symptom-ameliorating protein, or the payload may be an inhibitory RNA.

Eye imaging can be augmented or accomplished by detection of a marker delivered by the methods disclosed. The marker may be, for example fluorescent, radioactive, chromogenic, or enzymatic. Imaging techniques may be any known in the art, including without limitation scanning laser ophthalmoscope (SLO), scanning laser polarimeter, optical coherence tomography (OCT), ultrasound, MRI, and angiography. The detectable entity may be a nucleic acid, or a product produced by a transcribed protein, for example.

The nucleic acid may be transcribed to form transcripts and at least one of the transcripts may be translated to express a protein. In an alternative embodiment, the delivered nucleic acid itself is translated to express a protein. Thus the transcript encodes a therapeutic protein, preferably with sequence features necessary for transcription, such as a promoter, located in a suitable position relative to the coding sequence. Alternatively, the nucleic acid may be transcribed to form transcripts that are anti-sense to a deleterious endogenous transcript. The anti-sense transcript may inhibit synthesis of an endogenous protein which has a negative effect in the ocular disorder. For example, the anti-sense transcript may inhibit synthesis of an endogenous protein with a dominant negative mutation. In one embodiment, the anti-sense transcript may inhibit synthesis of an endogenous rhodopsin protein with a dominant negative mutation.

If the nucleic acid encodes a protein, it may be a protein that is a cytokine, a chemokine, a growth factor, an anti-angiogenesis factor, or an antibody or antibody fragment or construct. Particular proteins which may be used in the methods include, without limitation, ABCA4, MYO7A, ND4, GUCY2D, RPE65, Pigment epithelium-derived factor (PEDF), sFlt-1, ABCA; BEST; CORF; CA; CERKL; CHM; CLRN; CNGA; CNGB; CRB; CRX; DHDDS; EYS; FAMA; FSCN; GUCAB; IDHB; IMPDH; IMPG; KLHL; LRAT; MAK; MERTK; NRE; NRL; OFD; PDEA; PDEB; PDEG; PRCD; PROM; PRPF; PRPH; PRPH2; RBP; RDH; RGR; RHO; RLBP; ROM; RP; RPE; RPGR; RS1; SAG; SEMAA; SNRNP; SPATA; TOPORS; TTC; TULP; USHA; ZNF; ABHD12; CDH23; CIB2; CLRN1; DFNB31; GPR98; HARS; MYO7A; PCDH15; USH1C; USH1G; USH2A, ARL6; BBS1; BBS2; BBS4; BBS5; BBS7; BBS9; BBS10; BBS12; CEP290; INPP5E; LZTFL1; MKKS; MKS1; SDCCAG8; TRIM32; TTC8; endostatin, and angiostatin.

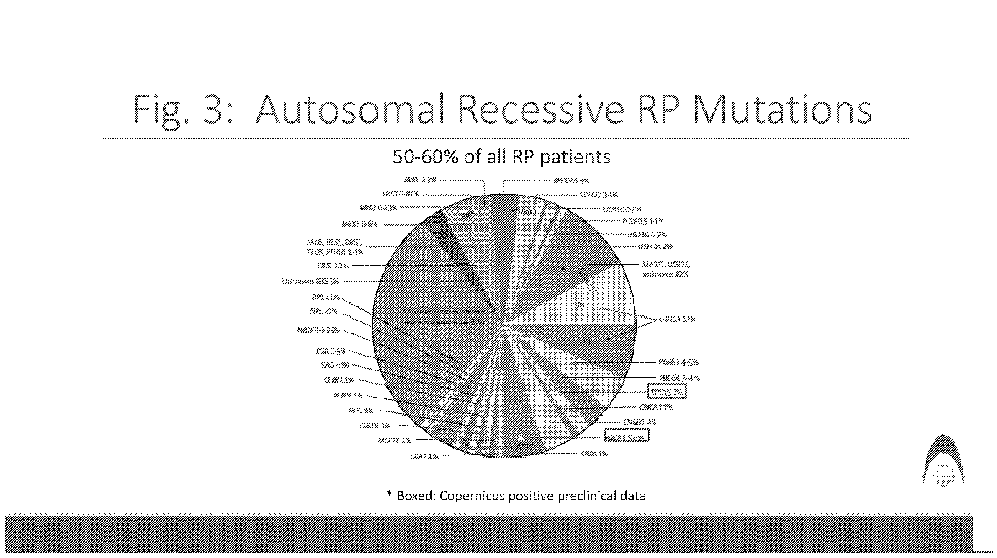

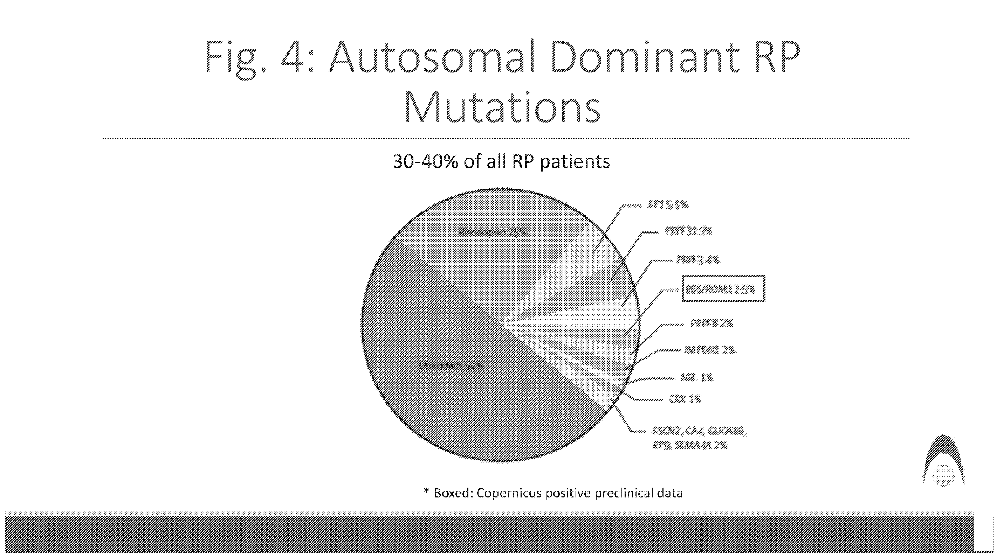

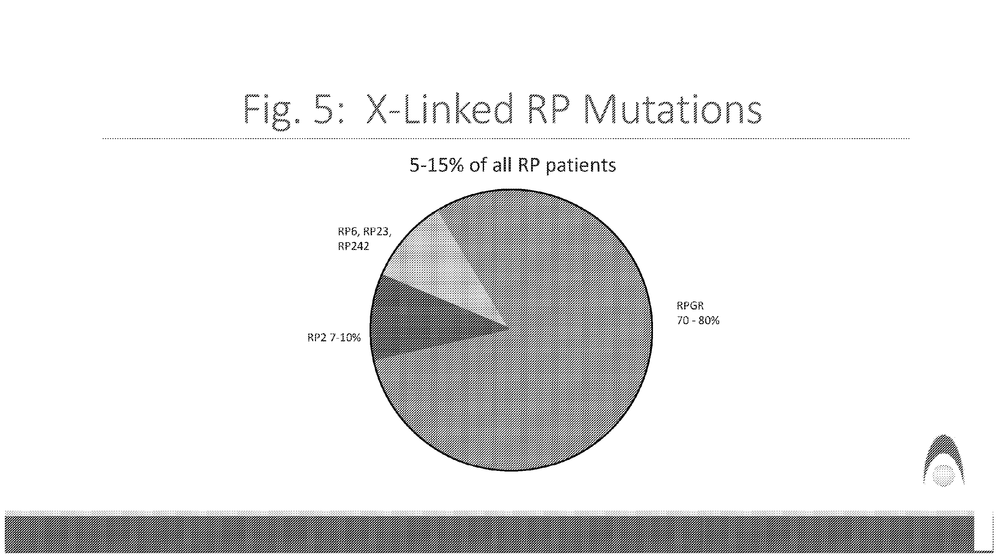

In some embodiments the nucleic acid encodes a wild-type form of a protein, a mutant form of which causes or exacerbates an ocular disease. In some embodiments the nucleic acid encodes a wild-type form of a protein, a mutant form of which causes or contributes to a genetic blinding disorder. In some embodiments the nucleic acid encodes a wild-type form of a protein, a mutant form of which causes or contributes to causation or severity of retinitis pigmentosa. Some genes which are mutated in retinitis pigmentosa are shown in Figs. 3-5. In some embodiments the ocular disease being treated is acquired, and in some it is inherited.

As used here, "non-surgical" ocular nucleic acid delivery methods refer to methods of nucleic acid delivery that do not require general anesthesia and/or retrobulbar anesthesia (also referred to as a retrobulbar block). Alternatively or additionally, a "non-surgical" ocular nucleic acid delivery method is performed with an instrument having a diameter of 28 gauge or smaller. Alternatively or additionally, "non-surgical" ocular nucleic acid delivery methods do not require a guidance mechanism that is typically required for ocular nucleic acid delivery via a shunt or cannula.

The non-surgical ocular disorder treatment methods described here are particularly useful for the local delivery of nucleic acids to the posterior region of the eye, for example the retinochoroidal tissue, macula, retinal pigment epithelium (RPE) and optic nerve in the posterior segment of the eye. In another embodiment, the non-surgical methods and microneedles provided here can be used to target nucleic acid delivery to specific posterior ocular tissues or regions within the eye or in neighboring tissue. In one embodiment, the methods described here deliver nucleic acid specifically to the sclera, the choroid, the Brach's membrane, the retinal pigment epithelium, the subretinal space, the retina, the macula, the optic disk, the optic nerve, the ciliary body, the trabecular meshwork, the aqueous humor, the vitreous humor, and/or other ocular tissue or neighboring tissue in the eye of a human subject in need of treatment. In one embodiment, the methods can be used to target nucleic acid delivery to specific posterior ocular tissues or regions within the eye or in neighboring tissue.

In one embodiment, the effective amount of the nucleic acid administered to the SCS provides higher therapeutic efficacy of the nucleic acid, compared to the therapeutic efficacy of the nucleic acid when the identical dosage is administered intravitreally, topically, intracamerally, parenterally or orally. In one embodiment, the microneedle nucleic acid delivery methods described here precisely deliver the nucleic acid into the SCS for subsequent local delivery to nearby posterior ocular tissues in need of treatment. The nucleic acid may be released into the ocular tissues from the infused formulation or from the nanoparticles over an extended period, *e.g.,* several hours or days or weeks or months, after the non-surgical nucleic acid administration has been completed. This beneficially can provide increased bioavailability of the nucleic acid relative, for example, to delivery by topical application of the nucleic acid formulation to ocular tissue surfaces, or increased bioavailability compared to oral, parenteral on intravitreal administration of the same nucleic acid dosage.

With the methods and microneedle devices described here, the SCS nucleic acid delivery methods advantageously include precise control of the depth of insertion into the ocular tissue, so that the microneedle tip can be placed into the eye so that the nucleic acid formulation flows into the suprachoroidal space and in some embodiments to the posterior ocular tissues surrounding the SCS. In one embodiment, insertion of the microneedle is in the sclera of the eye. In one embodiment, nucleic acid flow into the SCS is accomplished without contacting underlying tissues with the microneedle, such as choroid and retina tissues.

The methods provided here, in one embodiment, achieve delivery of nucleic acid to the suprachoroidal space, thereby allowing nucleic acid access to posterior ocular tissues not obtainable via topical, parenteral, intracameral or intravitreal nucleic acid delivery. Because the methods provided here deliver nucleic acid to the posterior ocular tissue for the treatment of a posterior ocular disorder or choroidal malady, the suprachoroidal nucleic acid dose sufficient to achieve a therapeutic response in a human subject treated with the methods provided here is less than the intravitreal, topical, parenteral or oral nucleic acid dose sufficient to elicit the same or substantially the same therapeutic response. In one embodiment, the SCS delivery methods described here allow for decreased nucleic acid dose of the posterior ocular disorder treating nucleic acid, or the choroidal malady treating nucleic acid, compared to the intravitreal, topical, intracameral parenteral or oral nucleic acid dose sufficient to elicit the same or substantially the same therapeutic response. In a further embodiment, the suprachoroidal nucleic acid dose sufficient to elicit a therapeutic response is 75% or less, or 50% or less, or 25% or less than the intravitreal, topical parenteral or oral nucleic acid dose sufficient to elicit a therapeutic response. The therapeutic response, in one embodiment, is a reduction in severity of a symptom/clinical manifestation of the ocular disorder, whether *e.g.,* a posterior ocular disorder or a choroidal malady, for which the patient is undergoing treatment, or a reduction in number of symptom(s)/clinical manifestation(s) of the posterior ocular disorder choroidal malady for which the patient is undergoing treatment.

The term "suprachoroidal space," is used interchangeably with suprachoroidal, SCS, suprachoroid and suprachoroidia, and describes the potential space in the region of the eye disposed between the sclera and choroid. This region primarily is composed of closely packed layers of long pigmented processes derived from each of the two adjacent tissues; however, a space can develop in this region as a result of fluid or other material buildup in the suprachoroidal space and the adjacent tissues. The "supraciliary space," is encompassed by the SCS and refers to the most anterior portion of the SCS adjacent to the ciliary body, trabecular meshwork and limbus. Those skilled in the art will appreciate that the suprachoroidal space frequently is expanded by fluid buildup because of some disease state in the eye or as a result of some trauma or surgical intervention. In the present description, however, the fluid buildup is intentionally created by infusion of a nucleic acid formulation into the suprachoroid to create the suprachoroidal space (which is filled with nucleic acid formulation). Not wishing to be bound by theory, it is believed that the SCS region serves as a pathway for uveoscleral outflow (*i.e.,* a natural process of the eye moving fluid from one region of the eye to the other through) and becomes a real space in instances of choroidal detachment from the sclera.

As used here, "ocular tissue" and "eye" include both the anterior segment of the eye (*i.e.,* the portion of the eye in front of the lens) and the posterior segment of the eye (*i.e.,* the portion of the eye behind the lens). The anterior segment is bounded by the cornea and the lens, while the posterior segment is bounded by the sclera and the lens. The anterior segment is further subdivided into the anterior chamber, between the iris and the cornea, and the posterior chamber, between the lens and the iris. The exposed portion of the sclera on the anterior segment of the eye is protected by a clear membrane referred to as the conjunctiva. Underlying the sclera is the choroid and the retina, collectively referred to as retinachoroidal tissue. The loose connective tissue, or potential space, between the choroid and the sclera is referred to as the suprachoroidal space (SCS). The cornea is composed of the epithelium, the Bowman's layer, the stroma, the Descemet's membrane, and the endothelium. The sclera with surrounding Tenon's Capsule or conjunctiva, suprachoroidal space, choroid, and retina, both without and with a fluid in the suprachoroidal space, respectively.

Devices and administration methods useful in the methods provided herein are known in the art, for example, in WO2017/192565, WO2014/179698, WO2014/074823, WO2011/139713, WO2007/131050, and WO2007/004874. The methods may be carried out with a hollow or solid microneedle, for example, a rigid microneedle. The term "microneedle" refers to a conduit body having a base, a shaft, and a tip end suitable for insertion into the sclera and other ocular tissue and has dimensions suitable for minimally invasive insertion and nucleic acid formulation infusion as described here, and as described in WO2017/192565, WO2014/179698, WO2014/074823, WO2011/139713, WO2007/131050, and WO2007/004874. In some embodiments, the microneedle has a length or effective length that does not exceed about 2000 microns and a diameter that does not exceed about 600 microns. Both the "length" and "effective length" of the microneedle encompass the length of the shaft of the microneedle and the bevel height of the microneedle.

The term "hollow" includes a single, straight bore through the center of the microneedle, as well as multiple bores, bores that follow complex paths through the microneedles, multiple entry and exit points from the bore(s), and intersecting or networks of bores. That is, a hollow microneedle has a structure that includes one or more continuous pathways from the base of the microneedle to an exit point (opening) in the shaft and/or tip portion of the microneedle distal to the base.

The microneedle device may further comprise a fluid reservoir for containing the nucleic acid formulation, *e.g.,* as a solution or suspension, and the nucleic acid reservoir being in operable communication with the bore of the microneedle at a location distal to the tip end of the microneedle. The fluid reservoir may be integral with the microneedle, integral with the elongated body, or separate from both the microneedle and elongated body.

The microneedle can be formed/constructed of different biocompatible materials, including metals, glasses, semi-conductor materials, ceramics, or polymers. Examples of suitable metals include pharmaceutical grade stainless steel, gold, titanium, nickel, iron, gold, tin, chromium, copper, and alloys thereof. The polymer can be biodegradable or non-biodegradable. Examples of suitable biocompatible, biodegradable polymers include polylactides, polyglycolides, polylactide-co-glycolides (PLGA), polyanhydrides, polyorthoesters, polyetheresters, polycaprolactones, polyesteramides, poly(butyric acid), poly(valeric acid), polyurethanes and copolymers and blends thereof. Representative non-biodegradable polymers include various thermoplastics or other polymeric structural materials known in the fabrication of medical devices. Examples include nylons, polyesters, polycarbonates, polyacrylates, polymers of ethylene-vinyl acetates and other acyl substituted cellulose acetates, non-degradable polyurethanes, polystyrenes, polyvinyl chloride, polyvinyl fluoride, poly(vinyl imidazole), chlorosulphonate polyolefins, polyethylene oxide, blends and copolymers thereof. Biodegradable microneedles can provide an increased level of safety compared to non-biodegradable ones, such that they are essentially harmless even if inadvertently broken off into the ocular tissue.

The microneedle can be fabricated by a variety of methods known in the art or as described in the example below. In one embodiment, the hollow microneedle is fabricated using a laser or similar optical energy source. In one example, a microcannula may be cut using a laser to represent the desired microneedle length. The laser may also be use to shape single or multiple tip openings. Single or multiple cuts may be performed on a single microcannula to shape the desired microneedle structure. In one example, the microcannula may be made of metal such as stainless steel and cut using a laser with a wavelength in the infrared region of the light spectrum (*e.g.,* from about 0.7 to about 300 µm). Further refinement may be performed using metal electropolishing techniques familiar to those in the field. In another embodiment, the microneedle length and optional bevel is formed by a physical grinding process, which for example may include grinding a metal cannula against a moving abrasive surface. The fabrication process may further include precision grinding, micro-bead jet blasting and ultrasonic cleaning to form the shape of the desired precise tip of the microneedle.

Further details of possible manufacturing techniques are described, for example, in U.S. Patent Application Publication No. 2006/0086689, U.S. Patent Application Publication No. 2006/0084942, U.S. Patent Application Publication No. 2005/0209565, U.S. Patent Application Publication No. 2002/0082543, U.S. Pat. No. 6,334,856, U.S. Pat. No. 6,611,707, U.S. Pat. No. 6,743,211.

The methods provided here allow for suprachoroidal nucleic acid delivery to be accomplished in a minimally invasive, non-surgical manner, superior to other non-surgical (*e.g.,* conventional needle) and surgical approaches. For instance, in one embodiment, the methods provided here are carried out via the use of one or more microneedles. In one embodiment, the microneedles are inserted perpendicular, or at an angle from about 80 degrees to about 100 degrees, into the eye, *e.g.,* into the sclera, reaching the suprachoroidal space in a short penetration distance. This is in contrast to long conventional needles or cannula which must approach the suprachoroidal space at a steep angle, taking a longer penetration path through the sclera and other ocular tissues, increasing the invasiveness of the method, the size of the needle track and consequently increasing the risk of infection and/or vascular rupture. With such long needles, the ability to precisely control insertion depth is diminished relative to the microneedle approach described here.

The microneedle, in one embodiment, is part of an array of two or more microneedles such that the method further includes inserting at least a second microneedle into the sclera without penetrating across the sclera. In one embodiment, where an array of two or more microneedles are inserted into the ocular tissue, the nucleic acid formulation of each of the two or more microneedles may be identical to or different from one another, in nucleic acid, formulation, volume/quantity of nucleic acid formulation, or a combination of these parameters. In one case, different types of nucleic acid formulations may be injected via the one or more microneedles. For example, inserting a second hollow microneedle comprising a second nucleic acid formulation into the ocular tissue will result in delivery of the second nucleic acid formulation into the ocular tissue.

In some embodiments, the microneedle devices employed here may be adapted to remove substances, such as a fluid, tissue, or molecule sample, from the eye. Those skilled in the art will appreciate, however, that other types of microneedles (*e.g.,* solid microneedles) and other methods of delivering the nucleic acid formulation into the suprachoroidal space and posterior ocular tissues may be used instead of or in conjunction with the delivery methods. Non-limiting examples include dissolving, at least in part, a coating of a nucleic acid formulation off of a microneedle; detaching, at least in part, a coating of a nucleic acid formulation (*e.g.,* as a substantially intact sleeve or in fragments) off of a microneedle; breaking or dissolving a microneedle off of a base to which the microneedle is integrally formed or is connected; or any combination thereof.

The mammals treated may be, for example, rabbit, primate, ungulate, bovine, porcine, canine, or human. A human subject treated may be an adult or a child. A wide range of ocular disorders, including posterior ocular disorders and choroidal maladies are treatable with the methods described here. Examples of posterior ocular disorders amenable for treatment by the methods include, but are not limited to, uveitis, glaucoma, macular edema, diabetic macular edema, retinopathy, age-related macular degeneration (for example, wet AMD or dry AMD), retinitis pigmentosa, juvenile onset macular degeneration, scleritis, optic nerve degeneration, geographic atrophy, choroidal disease, ocular sarcoidosis, optic neuritis, choroidal neovascularization, ocular cancer, genetic disease(s), autoimmune diseases affecting the posterior segment of the eye, retinitis (*e.g.,* cytomegalovirus retinitis) and corneal ulcers. The posterior ocular disorders amenable for treatment by the methods, devices, and nucleic acid formulations described here may be acute or chronic. For example, the ocular disease may be acute or chronic uveitis. Uveitis can be caused by infection with viruses, fungi, or parasites; the presence of noninfectious foreign substances in the eye; autoimmune diseases; or surgical or traumatic injury. Disorders caused by pathogenic organisms that can lead to uveitis or other types of ocular inflammation include, but are not limited to, toxoplasmosis, toxocariasis, histoplasmosis, herpes simplex or herpes zoster infection, tuberculosis, syphilis, sarcoidosis, Vogt-Koyanagi-Harada syndrome, Behcet's disease, idiopathic retinal vasculitis, Vogt-Koyanagi-Harada Syndrome, acute posterior multifocal placoid pigment epitheliopathy (APMPPE), presumed ocular histoplasmosis syndrome (POHS), birdshot chroidopathy, Multiple Sclerosis, sympathetic opthalmia, punctate inner choroidopathy, pars planitis, or iridocyclitis. Acute uveitis occurs suddenly and may last for up to about six weeks. Chronic uveitis is a form of uveitis in which the onset of signs and/or symptoms is gradual, and symptoms last longer than about six weeks.

Signs of uveitis include ciliary injection, aqueous flare, the accumulation of cells visible on ophthalmic examination, such as aqueous cells, retrolental cells, and vitreouscells, keratic precipitates, and hypema. Symptoms of uveitis include pain (such as ciliary spasm), redness, photophobia, increased lacrimation, and decreased vision. Posterior uveitis affects the posterior or choroid part of the eye. Inflammation of the choroid part of the eye is also often referred to as choroiditis. Posterior uveitis is may also be associated with inflammation that occurs in the retina (retinitis) or in the blood vessels in the posterior segment of the eye (vasculitis). In one embodiment, the methods provided here comprise non-surgically administering to a uveitis patient in need thereof, an effective amount of a uveitis treating nucleic acid to the SCS of the eye of the patient. In a further embodiment, the patient experiences a reduction in the severity of the symptoms, after administration of a uveitis treating nucleic acid to the SCS.

In one embodiment, the nucleic acid formulation delivered to the SCS results in the patient experiencing a reduction in inflammation, neuroprotection, complement inhibition, drusen formation, scar formation, and/or a reduction in choriocapillaris or choroidal neovascularization.

The non-surgical methods described here are particularly useful for the local delivery of nucleic acids to the posterior region of the eye, for example the retinochoroidal tissue, macula, and optic nerve in the posterior segment of the eye. In one embodiment, the non-surgical treatment methods and devices described here may be used in gene-based therapy applications. For example, the method, in one embodiment, comprises administering a nucleic acid formulation into the suprachoroidal space to deliver select DNA, RNA, or oligonucleotides to targeted ocular tissues.

The methods described here may be used for the treatment of a choroidal malady in a patient in need of such treatment. In one embodiment, the patient in need of choroidal malady treatment has been unresponsive to a previous non-SCS method for treating the choroidal malady. Examples of choroidal maladies amenable for treatment by the methods, devices and nucleic acid formulations described here include, but are not limited to, choroidal neovascularization, polypoidal choroidal vasculopathy, central sirrus choroidopathy, a multi-focal choroidopathy or a choroidal dystrophy (*e.g.,* central gyrate choroidal dystrophy, serpiginous choroidal dystrophy or total central choroidal atrophy). Choroidal maladies are described in further detail below.

In one embodiment, the choroidal malady treating nucleic acid has the effect of an angiogenesis inhibitor, a vascular permeability inhibitor or an anti-inflammatory agent, either by encoding a protein with such an activity or by inhibiting synthesis of a protein with the opposite effect. The angiogenesis inhibitor, in one embodiment, is a vascular endothelial growth factor (VEGF) modulator or a platelet derived growth factor (PDGF) modulator. The choroidal malady treatment method, in one embodiment, comprises administering the nucleic acid formulation to the SCS of one or both eyes of the patient in need of treatment via a microneedle. In a further embodiment, the microneedle is a hollow microneedle having a tip and an opening, and the nucleic acid formulation is infused into the SCS of one or both eyes through the tip of the hollow microneedle.

It should be noted that the desired infusion pressure to deliver a suitable amount of nucleic acid formulation might be influenced by the depth of insertion of the microneedle and the composition of the nucleic acid formulation. For example, a greater infusion pressure may be required in embodiments where the nucleic acid formulation for delivery into the eye is in the form of or includes nanoparticles encapsulating the active agent or microbubbles. In one embodiment, the nucleic acid formulation is comprised of nucleic acid particles in suspension with a D₉₉ of 30 nm or less. In one embodiment, the nucleic acid formulation is comprised of nucleic acid nanoparticles in suspension with a D₉₉ of 25 nm or less. In another embodiment, the nucleic acid formulation is comprised of nucleic acid particles in suspension with a D₉₉ of 20 nm or less.

In one embodiment, the non-surgical method of administering a nucleic acid to the SCS further includes partially retracting the hollow microneedle after insertion of the microneedle into the eye, and before and/or during the infusion of the nucleic acid formulation into the suprachoroidal space. In a particular embodiment, the partial retraction of the microneedle occurs prior to the step of infusing the nucleic acid formulation into the ocular tissue. This insertion/retraction step may form a pocket and beneficially permits the nucleic acid formulation to flow out of the microneedle unimpeded or less impeded by ocular tissue at the opening at the tip portion of the microneedle. This pocket may be filled with nucleic acid formulation, but also serves as a conduit through the nucleic acid formulation can flow from the microneedle, through the pocket and into the suprachoroidal space.

Targeting a nucleic acid formulation to the SCS and the posterior ocular tissues allows for high concentrations of the nucleic acid to be delivered to the choroid/sclera and the retina, with little to no nucleic acid being delivered to the aqueous humor of the anterior chamber. Additionally, the methods provided here allow for greater nucleic acid retention in the eye compared to other nucleic acid delivery methods, for example, a greater amount of nucleic acid is retained in the eye when delivered via the methods provided here as compared to the same dose delivered via intracameral, intravitreal, topical, parenteral or oral nucleic acid delivery methods. Accordingly, in one embodiment, the intraocular elimination half-life (t_{1/2}) of the nucleic acid when delivered via the methods described here is greater than the intraocular t_{1/2} of the nucleic acid when the same nucleic acid dose is administered intravitreally, intracamerally, topically, parenterally or orally. In another embodiment, the intraocular Cₘₐₓ of the nucleic acid, when delivered via the methods described here, is greater than the intraocular Cₘₐₓ of the nucleic acid when the same nucleic acid dose is administered intravitreally, intracamerally, topically, parenterally or orally. In another embodiment, the mean intraocular area under the curve (AUC₀₋ₜ) of the nucleic acid, when administered to the SCS via the methods described here, is greater than the intraocular AUC₀₋ₜ of the nucleic acid, when administered intravitreally, intracamerally, topically, parenterally or orally. In yet another embodiment, the intraocular time to peak concentration (tₘₐₓ) of the nucleic acid, when administered to the SCS via the methods described here, is greater than the intraocular tₘₐₓ of the nucleic acid, when the same nucleic acid dose is administered intravitreally, intracamerally, topically, parenterally or orally. In a further embodiment, the nucleic acid encodes or provides the function of an angiogenesis inhibitor, an anti-inflammatory nucleic acid (*e.g.,* a non-inflammatory cytokine), a VEGF modulator (*e.g.,* a VEGF antagonist), a PDGF modulator (*e.g.,* a PDGF antagonist), an immunosuppressive agent, or a vascular permeability inhibitor.

In one embodiment, the intraocular t_{1/2} of the nucleic acid when administered via the non-surgical SCS nucleic acid delivery methods provided here, is longer than the intraocular t_{1/2} of the nucleic acid when the identical dose is administered topically, intracamerally, intravitreally, orally or parenterally. In a further embodiment, the intraocular t_{1/2} of the nucleic acid when administered via the non-surgical SCS nucleic acid delivery methods provided here, is from about 1.1 times to about 10 times longer, or from about 1.25 times to about 10 times longer, or from about 1.5 times to about 10 times longer, or about 2 times to about 5 times longer, than the intraocular t_{1/2} of the nucleic acid when the identical dosage is administered topically, intracamerally, intravitreally, orally or parenterally. In a further embodiment, the nucleic acid encodes or provides the function of an angiogenesis inhibitor, an anti-inflammatory nucleic acid (*e.g.,* a non-inflammatory cytokine), a VEGF modulator (*e.g.,* a VEGF antagonist), a PDGF modulator (e.g., a PDGF antagonist), an immunosuppressive agent, or a vascular permeability inhibitor.

In another embodiment, the intraocular Cₘₐₓ of the nucleic acid, when delivered via the methods described here, is greater than the intraocular Cₘₐₓ of the nucleic acid when the same nucleic acid dose is administered intravitreally, intracamerally, topically, parenterally or orally. In a further embodiment, the intraocular Cₘₐₓ of the nucleic acid when administered via the non-surgical SCS nucleic acid delivery methods provided here, is at least 1.1 times greater, or at least 1.25 times greater, or at least 1.5 times greater, or at least 2 times greater, or at least 5 times greater, than the intraocular Cₘₐₓ of the nucleic acid when the identical dose is administered topically, intracamerally, intravitreally, orally or parenterally. In one embodiment, the intraocular C.sub.max of the nucleic acid when administered via the non-surgical SCS nucleic acid delivery methods provided here, is about 1 to about 2 times greater, or about 1.25 to about 2 times greater, or about 1 to about 5 times greater, or about 1 to about 10 times greater, or about 2 to about 5 times greater, or about 2 to about 10 times greater, than the intraocular Cₘₐₓ of the nucleic acid when the identical dose is administered topically, intracamerally, intravitreally, orally or parenterally. In a further embodiment, the nucleic acid encodes or provides the function of an angiogenesis inhibitor, an anti-inflammatory nucleic acid (*e.g.,* a non-inflammatory cytokine), a VEGF modulator (*e.g.,* a VEGF antagonist), a PDGF modulator (*e.g.,* a PDGF antagonist), an immunosuppressive agent or a vascular permeability inhibitor.

In another embodiment, the mean intraocular area under the curve (AUC₀₋ₜ) of the nucleic acid, when administered to the SCS via the methods described here, is greater than the intraocular AUC₀₋ₜ of the nucleic acid, when administered intravitreally, intracamerally, topically, parenterally or orally. In a further embodiment, the intraocular AUC₀₋ₜ of the nucleic acid when administered via the non-surgical SCS nucleic acid delivery methods provided here, is at least 1.1 times greater, or at least 1.25 times greater, or at least 1.5 times greater, or at least 2 times greater, or at least 5 times greater, than the intraocular AUC₀₋ₜ of the nucleic acid when the identical dose is administered topically, intracamerally, intravitreally, orally or parenterally. In one embodiment, the intraocular AUC₀₋ₜ of the nucleic acid when administered via the non-surgical SCS nucleic acid delivery methods provided here, is about 1 to about 2 times greater, or about 1.25 to about 2 times greater, or about 1 to about 5 times greater, or about 1 to about 10 times greater, or about 2 to about 5 times greater, or about 2 to about 10 times greater, than the intraocular AUC₀₋ₜ of the nucleic acid when the identical dose is administered topically, intracamerally, intravitreally, orally or parenterally. In a further embodiment, the nucleic acid encodes or provides the function of an angiogenesis inhibitor, an anti-inflammatory nucleic acid (*e.g.,* a non-inflammatory cytokine), a VEGF modulator (*e.g.,* a VEGF antagonist), a PDGF modulator (*e.g.,* a PDGF antagonist), an immunosuppressive agent or a vascular permeability inhibitor.

In one embodiment, the nucleic acid formulation comprising the effective amount of the nucleic acid, once delivered to the SCS, is substantially retained in the SCS over a period of time. For example, in one embodiment, about 80% of the nucleic acid formulation is retained in the SCS for about 30 minutes, or about 1 hour, or about 4 hours or about 24 hours or about 48 hours or about 72 hours. In this regard, a depot of nucleic acid is formed in the SCS and/or surrounding tissue, to allow for sustained release and/or cellular uptake of the nucleic acid over a period of time.

In one embodiment, the suprachoroidal space, once loaded with nucleic acid (*e.g.,* nucleic acid nanoparticles), provides a sustained release of nucleic acid to the retina or other posterior ocular tissues over a period of time. The targeting of the nucleic acid to the posterior ocular tissues via the methods described here allows for a greater therapeutic efficacy in the treatment of one or more posterior ocular disorders or choroidal maladies (*e.g.,* PCV), as compared to other administration methods of the same nucleic acid dose, such as intravitreal, intracameral, oral, parenteral and topical delivery of the same nucleic acid dose. In a further embodiment, the therapeutic effect of the nucleic acid delivered to the SCS is achieved with a lower dose than the intravitreal, intracameral, topical, parenteral or oral dose sufficient to achieve the same therapeutic effect in the human subject. Additionally, without wishing to be bound by theory, the lower doses achievable with the methods provided here result in reduced number of side effects of the nucleic acid, and/or reduced severity of one or more side effect(s), compared to higher doses of the nucleic acid, or the same nucleic acid dose delivered to the human patient via non-suprachoroidal routes of administration (*e.g.,* intravitreal, intracameral, topical, parenteral, oral). For example, the methods provided here provide a reduced number of side effects, or reduced severity of one or more side effects, or clinical manifestations, as compared to oral, topical, intracameral, parenteral or intravitreal administration of the same nucleic acid at the same dose. In one embodiment, the side effect or clinical manifestation that is lessened in the treated patient is subretinal exudation and/or subretinal bleeding.

In one embodiment, the non-surgical suprachoroidal nucleic acid delivery methods provided here result in an increased therapeutic efficacy and/or improved therapeutic response, as compared to oral, parenteral and/or intravitreal nucleic acid delivery methods of the identical or similar nucleic acid dose. In one embodiment, the SCS nucleic acid dose sufficient to provide a therapeutic response is about 90%, or about 75%, or about one-half (*e.g.,* about one half or less) the intravitreal, intracameral, topical, oral or parenteral nucleic acid dose sufficient to provide the same or substantially the same therapeutic response. In another embodiment, the SCS dose sufficient to provide a therapeutic response is about one-fourth the intravitreal, intracameral, topical, oral or parenteral nucleic acid dose sufficient to provide the same or substantially the same therapeutic response. In yet another embodiment, the SCS dose sufficient to provide a therapeutic response is one-tenth the intravitreal, intracameral, topical, oral or parenteral nucleic acid dose sufficient to provide the same or substantially the same therapeutic response. In one embodiment, the therapeutic response is a decrease in inflammation, as measured by methods known to those of skill in the art. In another embodiment, the therapeutic response is a decrease in number of ocular lesions, or decrease in ocular lesion size.

In one embodiment, the nucleic acid which is compacted is selected from a suitable oligonucleotide (e.g., antisense oligonucleotide agents), polynucleotide (*e.g.,* therapeutic DNA), ribozyme, dsRNA, siRNA, RNAi, gene therapy vectors, and/or vaccine. In a further embodiment, the nucleic acid is an aptamer (*e.g.,* an oligonucleotide or peptide molecule that binds to a specific target molecule). In another embodiment, the nucleic acid formulation delivered via the methods provided here encodes an endogenous protein or fragment thereof, or an endogenous peptide or fragment thereof. In one embodiment, the non-surgical treatment methods and devices described here may be used in gene-based therapy applications. For example, the method, in one embodiment, comprises administering a fluid nucleic acid formulation into the suprachoroidal space to deliver select DNA, RNA, or oligonucleotides to targeted ocular tissues.

In one embodiment, the nucleic acid which is compacted is useful in treating a choroidal malady. In a further embodiment, the choroidal malady treating nucleic acid is a nucleic acid administered to inhibit gene expression. For example, the nucleic acid, in one embodiment, is a micro-ribonucleic acid (microRNA), a small interfering RNA (siRNA), a small hairpin RNA (shRNA) or a double stranded RNA (dsRNA), that targets a gene involved in angiogenesis. In one embodiment, the methods provided here to treat a choroidal malady comprise administering an RNA molecule to the SCS of a patient in need thereof. In a further embodiment, the RNA molecule is delivered to the SCS via one of the microneedles described here. In one embodiment, the patient is being treated for PCV, and the RNA molecule targets HTRA1, CFH, elastin or ARMS2, such that the expression of the targeted gene is down-regulated in the patient, upon administration of the RNA. In a further embodiment, the targeted gene is CFH, and the RNA molecule targets a polymorphism selected from rs3753394, rs800292, rs3753394, rs6680396, rs1410996, 84664, rs1329428, and rs1065489. In another embodiment, the patient is being treated for a choroidal dystrophy, and the RNA molecule targets the PRPH2 gene. In a further embodiment, the RNA molecule targets a mutation in the PRPH2 gene. The nucleic acid delivered to the suprachoroidal space via the non-surgical methods described here, is present as a nucleic acid formulation. The "nucleic acid formulation" in one embodiment, is an aqueous solution or suspension, and comprises an effective amount of the nucleic acid. Accordingly, in some embodiments, the nucleic acid formulation is a fluid nucleic acid formulation. The "nucleic acid formulation" is a formulation of a nucleic acid, which typically includes one or more pharmaceutically acceptable excipient materials known in the art. The term "excipient" refers to any non-active ingredient of the formulation intended to facilitate handling, stability, dispersibility, wettability, release kinetics, and/or injection of the nucleic acid. In one embodiment, the excipient may include or consist of water or saline.

The nucleic acid formulation (*e.g.,* fluid nucleic acid formulation) includes nanoparticles, which include just one nucleic acid molecule. Desirably, the nanoparticles provide for the release of nucleic acid into the suprachoroidal space and surrounding posterior ocular tissue. "Nanoparticles" are particles having an average diameter of from about 1 nm to about 100 nm. In another embodiment, the D₅₀ of the particles in the nucleic acid formulation is about 100 nm or less. In another embodiment, the D₅₀ of the particles in the nucleic acid formulation is about 15 nm to about 30 nm, preferably 20 nm or less.

Nanoparticles may or may not be spherical in shape. They may be, *e.g.,* ellipsoid or rod shaped. The nucleic acid-containing nanoparticles may be suspended in an aqueous or non-aqueous liquid vehicle. The liquid vehicle may be a pharmaceutically acceptable aqueous solution, and optionally may further include a surfactant. The nanoparticles of nucleic acid themselves may include an excipient material, such as a polymer, a polysaccharide, a surfactant, etc., which are known in the art to control the kinetics of nucleic acid release from particles.

The above disclosure generally describes the present invention. A more complete understanding can be obtained by reference to the following specific examples which are provided here for purposes of illustration only, and are not intended to limit the scope of the invention, which is defined by the appended claims.

### EXAMPLE 1

The present invention is further illustrated by reference to the following examples. However, it should be noted that these Examples, like the embodiments described above, are illustrative and are not to be construed as restricting the scope of the invention in any way. The scope of the invention is defined by the appended claims.

### Materials and Methods

Unless otherwise specified, hollow microneedles were fabricated from borosilicate micropipette tubes (Sutter Instrument, Novato, Calif.), as described previously (J. Jiang, et al., Pharm. Res. 26:395-403 (2009)). A custom, pen-like device with a threaded cap was fabricated to position the microneedle and allow precise adjustment of its length. This device was attached to a micropipette holder (MMP-KIT, World Precision Instruments, Sarasota, Fla.) with tubing that was connected to a carbon dioxide gas cylinder for application of infusion pressure. The holder was attached to a micromanipulator (KITE, World Precision Instruments) which was used to control insertion of the microneedle into the sclera.

A custom acrylic mold, shaped to fit a whole eye, was built to hold the eye steady and used for all experiments. A catheter was inserted through the optic nerve into the vitreous and connected to a bottle of BSS Plus raised to a height to generate internal eye pressure (18 or 36 mm Hg). Suction was applied to a channel within the mold to hold the external surface of the eye steady during microneedle insertion and manipulation. Each microneedle was pre-filled with a desired volume of the material to be injected. The microneedle was placed in the device holder at a set microneedle length, attached to the micromanipulator and connected to the constant pressure source. Microneedles were then inserted perpendicular to the sclera tissue 5-7 mm posterior from the limbus. A set pressure was applied to induce infusion. Thirty seconds were allowed to see if infusion of the solution began. If infusion occurred, the pressure was stopped immediately upon injection of the specified volume. If visual observation of the injected material showed localization in the suprachoroidal space, the injection was considered a success. If infusion had not begun within that timeframe, then the applied pressure was stopped and the needle was retracted. This was considered an unsuccessful delivery.

Eyes to be imaged using microscopy were detached from the set-up within minutes after delivery was completed. The eyes were placed in acetone or isopentane kept on dry ice or liquid nitrogen, causing the eye to freeze completely within minutes after placement. The frozen eye was removed from the liquid and portions of the eye were hand cut using a razor blade for imaging of injected material. Imaging was performed using a stereo microscope using brightfield and fluorescence optics (model SZX12, Olympus America, Center Valley, Pa.). The portions containing the sclera, choroid and retina were placed in Optimal Cutting Temperature media (Sakura Finetek, Torrance, Calif.) and frozen under dry ice or liquid nitrogen. These samples were cryosectioned 10-30 .mu.m thick (Microm Cryo-Star HM 560MV, Walldorf, Germany) and imaged by brightfield and fluorescence microscopy (Nikon E600, Melville, N.Y.) to determine the location of injected material in the eye. Images were collaged as necessary using Adobe Photoshop software (Adobe Systems, San Jose, Calif.).

### EXAMPLE 2

### Safety, Tolerability, and Gene Transfer Study after Suprachoroidal (SC) Delivery of Non-Viral Nanoparticles in Rabbit

Objective: To evaluate the safety, tolerability, and the retinal cell types transfected after nanoparticle delivery in a short term (1-week) study following suprachoroidal administration as the delivery route of non-viral nanoparticles encoding two types of reporter genes.
Animals: Species / Strain: Rabbits / New Zealand White, Adult, Male
Number: 24
Regulatory status: Non-GLP

Treatments:

Test Articles: Test articles (TA) consist of non-viral ellipsoid or rod shaped DNA nanoparticles encoding either luciferase or eGFP reporter genes.

Controls were vehicle injected, uninjected eyes, and a positive control.

### Dosing:

Rabbits in group 1 received a single 100 µL injection of vehicle control (saline) via the suprachoroidal (SC) route; rabbits in groups 2-5 received a single 100 µL injection of TA via the SC route; rabbits in groups 6-7 served as positive controls, receiving 50 µL TA injected via the sub-retinal (SR) route. All treatments were administered to the OS; the OD remained untreated.)

### Experimental Design

| **Group ID** | **No. of Animals** | **Test article /route** | **End point parameters** | **Time of euthanasia** |
|---|---|---|---|---|
| 1 | 4 | OS : vehicle (100 µL)/SC | • OEs at baseline, 24 h post-injection and at harvest. | 1-week |
| | | | • IOP at baseline, at 24 h, and weekly until harvest. | |
| | | OD: none | ERG at baseline, and at harvest. | |
| | | | • eGFP IHC and Luciferase expression (via sponsor) | |
| 2 | 4 | OS : Active TA (ellipsoid luciferase) (100 µL)/SC | • OEs at baseline, 24 h post-injection and at harvest. | |
| | | | • IOP at baseline, at 24 h, and weekly until harvest. | |
| | | OD: none | • ERG at baseline, and at harvest. | |
| | | | • Luciferase expression (via sponsor) | |
| 3 | 4 | OS : Active TA (rod luciferase) (100 µL)/SC | • OEs at baseline, 24 h post-injection and at harvest. | 1-week |
| | | | • IOP at baseline, at 24 h, and weekly until harvest. | |
| | | OD: none | • ERG at baseline, and at harvest. | |
| | | | • Luciferase expression (via sponsor) | |
| 4 | 4 | OS : Active TA (ellipsoid eGFP)/ (100 µL)/SC | • OEs at baseline, 24 h post-injection and at harvest. | |
| | | | • IOP at baseline, at 24 h, and weekly until harvest. | |
| | | OD: none | • ERG at baseline, and at harvest. | |
| | | | • eGFP expression (via IHC) | |

| | | | | |
|---|---|---|---|---|
| 5 | 4 | OS : Active TA (rod cGFP)/(100 µL)/SC | • OEs at baseline, 24h post-injection and at harvest. | |
| | | | • IOP at baseline, at 24h, and weekly until harvest. | |
| | | OD: none | • ERG at baseline, and at harvest. | |
| | | | • eGFP expression (via IHC) | |
| 6 | 4 | OS : Positive Controls (rod luciferase) (50 µL)/SR | • OEs at baseline, 24h post-injection and at harvest. | |
| | | | • IOP at baseline, at 24h, and weekly until harvest. | |
| | | OD: none | • ERG at baseline, and at harvest. | |
| | | | • Luciferase expression (via sponsor) | |
| 7 | 4 | OS : Positive Controls (rod eGFP) (50 µL)/SR | • OEs at baseline, 24h post-injection and at harvest. | |
| | | | • IOP at baseline, at 24h, and weekly until harvest. | |
| | | OD: none | • ERG at baseline, and at harvest. | |
| | | | • eGFP expression (via IHC) | |

### Test System: Animals, Housing, and Environmental Conditions

| | |
|---|---|
| **Species/Strain** | Rabbit *(Oryctolagus cuniculus)*/New Zealand White |
| **Source** | Covance, Denver, PA |
| **Age Range at First Dosing** | Approximately 4-6 months |
| **Weight Range at First Dosing** | 2-3kg |
| **Identification** | Cage card |
| **Physical Examination Time** | During acclimation |
| **Caging** | Stainless steel; 17 inches wide x 27 inches deep x 15 inches tall or larger, slatted bottoms. No additional bedding. |
| **Number per cage** | 1 |
| **Environmental Conditions** | Photoperiod: 12 hrs light/12 hrs darkness |
| | Temperature: 68 ± 2°F |

### Animal Diet and Water:

| | | |
|---|---|---|
| **Feed** | **Type** | Hi Fiber Rabbit Diet |
| | **Name** | Hi Fiber Lab Rabbit Diet #5P25, Purina, St. Louis, MO |
| | **Availability** | *ad libitum* |
| | **Analysis for Contaminants** | Not routinely performed, No contaminants expected |
| **Water** | **Source** | Durham City Water |
| | **Availability** | *ad libitum* via water bottles with sipper tubes. |
| | **Analysis for Contaminants** | Every 6 months, No contaminants found |

### Animal Health and Acclimation:

Animals were acclimated to the study environment for a minimum of 2 weeks prior to anesthesia. At the completion of the acclimation period, each animal was physically examined by a laboratory animal technician for determination of suitability for study participation. Examinations included, but were not limited to, the skin and external ears, eyes, abdomen, neurological, behavior, and general body condition. Animals determined to be in good health were released to the study.

### Randomization and Study Identification:

Animals were assigned to study groups according to Powered Research Standard Operating Procedures(SOPs). Specifically, animals were assigned to groups by a stratified randomization scheme designed to achieve averaged mean weight in each group. Animals were uniquely identified by corresponding cage card number and ear tagging.

### Test Formulation and dosing:

Test articles (TA) were non-viral vectors, either ellipsoid or rod shaped nanoparticles, encoding either the luciferase or eGFP genes. The rabbits were given buprenorphine 0.01-0.05 mg/kg SQ. Rabbits were then tranquilized for the injections and the eyes aseptically prepared using topical 5% betadine solution, followed by rinsing with sterile eye wash, and application of one drop of proparacaine HCL and phenylephrine HCL. An eyelid speculum was placed, and vehicle control and TA were administered by suprachoroidal (SC) injections using a 30-gauge needle approximately 1000 µm in length (Clearside microinjector). Only the positive controls (luciferase and eGFP rod nanoparticles) were injected through the sub-retinal (SR) space in a 50 µl volume. Following the injection procedure, 1 drop of Neomycin Polymyxin B Sulfates Gramicidin ophthalmic solution was applied topically to the ocular surface.

### Parameters to be Measured:

### Examinations:

A veterinary ophthalmologist performed complete ocular examinations using a slit lamp biomicroscope and indirect ophthalmoscope to evaluate ocular surface morphology and anterior segment inflammation on all animals prior to injection to serve as a baseline as well as 24 hours post injection and at harvest. The Hackett and McDonald ocular grading system was used for scoring. Animals were not tranquilized for the examinations. (Hackett, R.B. and McDonald, T.O. Ophthalmic Toxicology and Assessing Ocular Irritation. Dermatoxicology, Fifth Edition. Ed. F.N. Marzulli and H.I. Maibach. Washington, D.C.: Hemisphere Publishing Corporation. 1996; 299-305 and 557-566.)

### Tonometry:

Intraocular pressure (IOP) was measured in both eyes prior to injections (baseline), at 24 h, then weekly until harvest. The measurements were taken using a Tonovet probe (iCare Tonometer, Espoo, Finland) without use of topical anesthetic. The tip of the Tonovet probe was directed to gently contact the central cornea. The average IOP shown on the display was recorded. This procedure was then repeated two additional times and the measurements were recorded and averaged.

### Electroretinography (ERG)

ERGs were done on both eyes of the rabbits at baseline and before euthanasia. All animals were dark adapted for at least 15 minutes prior to ERG. ERGs were elicited by brief flashes at 0.33 Hz delivered with a mini-ganzfeld photostimulator (Roland Instruments, Wiesbaden, Germany) at maximal intensity. Twenty responses were amplified, filtered, and averaged (Retiport Electrophysiologic Diagnostic Systems, Roland Instruments, Wiesbaden, Germany) for each animal.

### Ocular Histopathology:

At 1-week post-injection, OS and OD were enucleated immediately after euthanasia, fixed in Davidson Fixative and, after 24 h, tissue was transferred to 70% ethanol, and embedded in paraffin for sectioning. Sections were stained with hematoxylin and eosin (H&E) and anti-eGFP antibody.

### Ocular Dissection for Luciferase Assay:

At 1-week post-injection, OS and OD eyes were enucleated immediately after euthanasia. Aqueous humor was collected to depressurize the eyes and the globe was flash frozen. Retina and choroid were dissected from each eye while frozen and placed in preweighed tubes. The tubes were then weighed to determine the tissue weight and immediately placed on dry ice until transfer to a -80°C freezer. Frozen samples were stored at -80°C until assayed for luciferase activity.

### Justification:

This study was designed to determine the short and long term tolerability following SCS delivery of TA. The number of animals, data collection time points and parameters for measurement were chosen based on the minimum required to meet the objectives of the study.

### IACUC Compliance / Pain control:

The protocol was approved by the Powered Research IACUC. According to the IACUC and facility SOPs, cage-side examinations were done at least every 12 hours for signs of overt discomfort such as severe blepharospasm, severe conjunctival hyperemia, epiphora, excessive rubbing at the eye, and not eating. If these conditions persist for 12 hours then the rabbits were euthanized humanely.

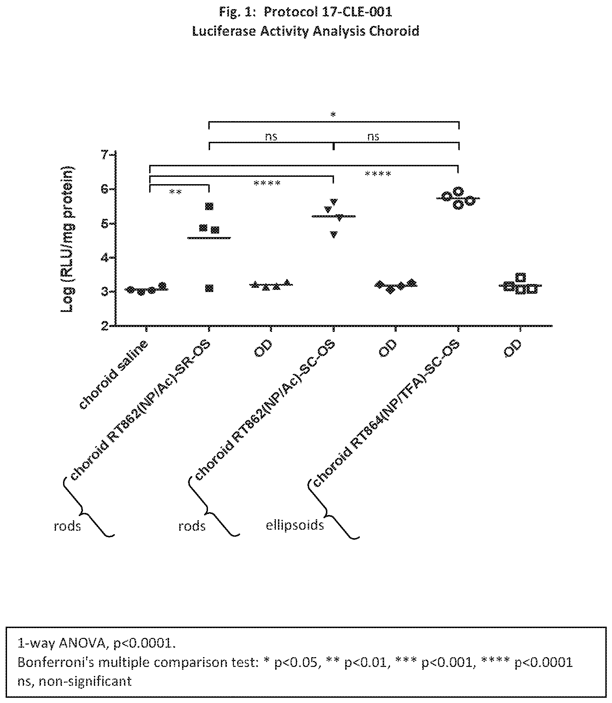

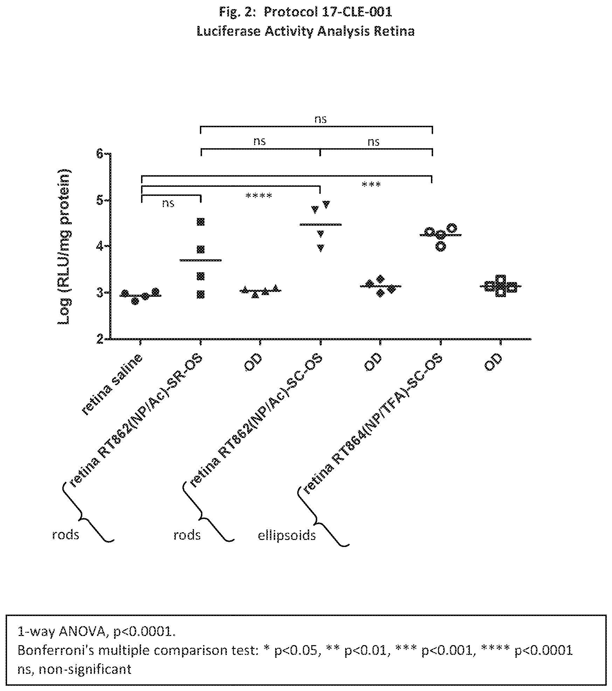

Results are shown in Figs. 1-2.

### EXAMPLE 3

### Three-Week Ocular Gene Delivery Study Following Suprachoroidal Administration of Luciferase-DNA Non-Viral Nanoparticle Formulations to Cynomolgus Monkeys

### OBJECTIVE

The purpose of this study is to assess the ocular gene delivery for three weeks after suprachoroidal administration of Luciferase DNA-containing non-viral nanoparticle formulations to cynomolgus monkeys.

### REGULATORY COMPLIANCE

This study will be conducted in accordance with the applicable standard operating procedures (SOPs). This study is not considered to be within the scope of the Good Laboratory Practice Regulations. All procedures in the Protocol are in compliance with the Animal Welfare Act Regulations (9 CFR 3).

Portions of the study conducted by OSOD will be in accordance with the applicable SOPs, the Protocol, any Protocol Amendments, and study-specific procedures, as applicable.

### Major Computer Systems

The major validated computer systems to be used on this study may include, but not be limited to, the following:

| System | Function |
|---|---|
| Electronic Notes (eNotes) | Documents study-specific communications |
| Pristima | Direct on-line capture of in-life data |
| Tox Reporting | Transfers data from Pristima for reporting purposes |
| Debra | An automated data capture and management system for data collection from balances |
| Documentum | Document management system for generation of study-related documents and electronic signatures |
| Metasys | An environmental monitoring system (EMS) for the animal facility |
| REES | An EMS for storage units |

### TEST ARTICLE FORMULATIONS

| | |
|---|---|
| Test article: | Luciferase ellipsoid Nanoparticle (NP) in saline |
| Storage conditions: | Approximately 5°C |
| Test article: | Luciferase rod NP in saline |
| Storage conditions: | Approximately 5°C |
| Control article: | Saline |
| Storage conditions: | Approximately 5°C |

### Purity

The chemical purity of the formulations is the responsibility of the Sponsor.

### Stability

Stability of the formulations is the responsibility of the Sponsor.

### Safety Precautions

Personnel will follow all safety precautions as required by Covance Policies and Procedures in consideration of the Safety Data Sheet or other relevant safety information provided by the Sponsor.

### Study Design:

| Group | Number of Female Animals | Dose Route | Formulation | Target Dose Level (mg DNA/eye) | Target Dose Volume (µL/eye) | Samples Collected |
|---|---|---|---|---|---|---|
| 1 | 1 | Suprachoroidal | Saline | NA | 100 | Ocular tissues |
| 2 | 4 | Suprachoroidal | Luciferase ellipsoid NP | 0.4 | 100 | Ocular tissues |
| 3 | 4 | Suprachoroidal | Luciferase rod NP | 0.4 | 100 | Ocular tissues |

| | | | | | | |
|---|---|---|---|---|---|---|
| Notes: Animals will receive a single dose to both eyes. Additional animals may be dosed for use as replacements in the event of a misdose or other unforeseen event, as applicable. | | | | | | |

### Animals and Husbandry

### Species

Primate

### Number and Sex

Nine females on test

### Strain and Source

Drug naive Cynomolgus monkey from Covance Research Products Inc., Alice, Texas

### Acclimation

Upon arrival, animals will be acclimated, maintained, and monitored for good health in accordance with SOPs or at the discretion of the Department of Animal Welfare and Comparative Medicine. Animals will be acclimated to the study room for at least one week prior to dose administration.

### Weight at Dose Administration

2 to 5 kg or greater

### Age at Dose Administration

2 to 7 years

### Housing

During acclimation and the test period, animals will be housed in stainless steel cages.

Animals will be commingled, as applicable, in accordance with Covance SOPs; animals will not be commingled for at least 24 hours after test article administration to allow monitoring of any test article-related effects. Animals may be individually housed for study-related procedures or behavioral or health reasons.

### Feed

Certified Primate Diet #5048 (PMI, Inc.) or #5L4L (PMI, Inc.) will be provided in accordance with SOPs.

### Water

Ad libitum, provided fresh daily

### Contaminants

There are no known contaminants in the food or water that would interfere with this study.

### Enrichment and Treats

For environmental and psychological enrichment, various cage and/or food enrichment (that do not require analysis) may be offered in accordance with the applicable SOPs. Diets may be supplemented with appropriate treats (that do not require analysis) in accordance with Covance SOPs.

### Environment

Environmental controls for the animal room will be set to maintain a temperature of 20 to 26°C, a relative humidity of 50 ± 20%, and a 12-hour light/12-hour dark cycle. The 12-hour dark cycle may be interrupted to accommodate study procedures.

### Animal Selection

Animals will not be randomized. Animals will be selected for use on test based on overall health, body weight, results of ophthalmic examinations, or other relevant data, as appropriate.

### Identification

Animals will be identified via individual cage cards, ear tag, tattoo, and/or implantable microchip identification devices (IMID), as applicable.

### Justification

The primate is a suitable species for evaluating ocular distribution; this model can also provide quantitative ocular distribution data. The number of animals is the minimum number required to obtain scientifically valid results and to ensure adequate sample size for analysis. In the opinion of the Sponsor and Study Director, this study does not unnecessarily duplicate previous work.

### Veterinary Care and Treatment

In accordance with the Animal Welfare Act, the Guide for the Care and Use of Laboratory Animals, and the Office of Laboratory Animal Welfare, medical treatment necessary to prevent unacceptable pain and suffering, including euthanasia, is the sole responsibility of the attending laboratory animal veterinarian. Discretionary medical treatment may be carried out based upon consensus agreement between the Study Director and the attending laboratory animal veterinarian. The Sponsor will be notified of any veterinary treatment.

### Reason for Dosing Route

The objective of the study is to evaluate the ocular gene delivery of non-viral nanoparticles containing Luciferase-DNA formulations. Suprachoroidal injection is the intended dose route in humans.

### Dose Preparation and Analysis

The dose formulations will be administered as provided by the Sponsor.

To prepare the formulations for administration to the animals, the vials will be allowed to come to ambient temperature and agitated by flicking the tube; do not vortex.

Hamilton syringes (provided by the Sponsor) fitted with 19-g needle will be filled with approximately 150 µL of formulation in a sterile laminar flow biosafety cabinet under aseptic conditions. This needle, used to transfer the formulation to the syringe, will be replaced with a 30 gauge luer-lock needle (700 µm), and the needles primed and capped for transport to the dosing room for use within 3 hours of filling.

Analysis of the dose formulations is the responsibility of the Sponsor.

### Dosing Procedures

Animals will not be fasted prior to dose administration.

### Analgesia Prior to and following Eye Preparation and/or Dosing

Analgesic agents will be administered as deemed necessary following eye preparation and/or dosing. Compounds to be used may include, but not be limited to the following: flunixin meglumine and buprenorphine.

### Anesthesia

Animals will be anesthetized by using the standard regimen of ketamine and dexmedetomidine. Inhalation anesthetic will also be administered if appropriate. Additional (or alternative) anesthetics and analgesics may be administered at the recommendation of the veterinary staff. All anesthetic and analgesic agents administered will be recorded in the data.

### Eye Preparation

Following application of topical anesthetic, eyes will be rinsed with an iodine solution for approximately 2 minutes followed by a saline rinse.

### Dose Administration

Following an injection site preparation, a single suprachoroidal injection of 100 µL given over 5 to 10 seconds will be administered to each eye (approximately 4 mm from the limbus, in the superior temporal quadrant) by an OSOD representative according to a study-specific procedure. Following the injection, the needle will be kept in the eye for approximately 10 seconds before being withdrawn. Upon withdrawal of the microneedle, a cotton-tipped applicator (CTA, dose wipe) will be placed over the injection site for approximately 5 seconds; the dose wipe will be discarded. The right eye will be dosed first; all postdose times will be based on the time of dosing of the second (left) eye.

Dosing observations will be recorded.

### Observation of Animals

### Antemortem Observations

On the day of arrival, animals will be observed for mortality and signs of pain and distress at least once, and cageside observations may be done for general health and appearance. Beginning the day after arrival, animals will be observed for mortality and signs of pain and distress at least twice daily (a.m. and p.m.), and cageside observations for general health and appearance will be done once daily. Additional observations may be conducted and any unusual observations will be recorded in the raw data.

### Body Weights

Body weights will be taken within 5 days of arrival and weekly throughout acclimation, as applicable. Animals will also be weighed at the time of animal selection, on the day of dose administration, and weekly throughout the remainder of the study, as applicable.

Additional body weights may be taken if necessary.

### STUDY ACTIVITIES

| | |
|---|---|
| Ophthalmic Observations: | Modified Hackett McDonald (One Round) |
| No. of Animals | All available |
| Frequency | Predose, 2 to 3 hours postdose, and on Study Days 8 and 22 |
| | Unscheduled ophthalmic examinations may be conducted if deemed necessary by the study director or veterinary ophthalmologist. |
| Conducted by | A veterinary ophthalmologist |
| Observations | Both eyes will be dilated with a mydriatic agent, then examined using a slitlamp biomicroscope and indirect ophthalmoscope. |
| | Both eyes will be grossly examined and graded using a modified Hackett-McDonald Scoring System (as seen in Attachment No. 1), with the following assessments excluded: pupillary light reflex and corneal fluorescein staining will only be performed at the discretion of the examining veterinary ophthalmologist. |
| | Abnormalities or an indication of normal will be recorded. At the discretion of the veterinary ophthalmologist, the eyes may be examined using other appropriate instrumentation. |

### SAMPLE COLLECTION

### Ocular Tissues

One animal in Group 1 will be sacrificed on Study Day 8. Two animals/group in
Groups 2 and 3 will be sacrificed on Study Days 8 and 22. Animals will be sacrificed via overdose of sodium pentobarbital. Blood will be collected via cardiac puncture to facilitate the collection of eyes and discarded; the volume of blood will not be recorded. At the time of sacrifice, both eyes will be enucleated followed by collection of the corneal epithelium and removal of aqueous humor (discarded), and flash frozen in liquid nitrogen for 15 to 20 seconds. The enucleated eye will be placed on dry ice or stored at approximately -70°C for at least two hours. Within approximately 5 days, the frozen matrices will be collected as right and left eye for each matrix into the specific tube type listed.

| | |
|---|---|
| Fresh Collection | Collection Tube Requirements |
| Corneal epithelium | 2-mL polypropylene Sarstedt tubes |
| *Frozen Collection* | |
| Choroid-retinal pigmented epithelium (RPE) | 2-mL polypropylene Sarstedt tubes |
| Ciliary body | 2-mL polypropylene Sarstedt tubes |
| Iris | 2-mL polypropylene Sarstedt tubes |
| Retina^{a} | 2-mL polypropylene Sarstedt tubes |
| a Filter paper will be used to collect retina. | |

The ocular tissues will be rinsed with saline and blotted dry, as appropriate, weighed, and placed on dry ice. All ocular tissues will be collected as single samples. Remaining ocular tissues will be discarded.

### Sample Identification and Storage

Samples will be uniquely identified to indicate origin and collection time. Sample storage will be as follows:

| Matrix | Storage Conditions | Comments |
|---|---|---|
| Ocular tissues | -70°C | Dry ice until stored at -70°C. |

| | | |
|---|---|---|
| Note: Temperatures are approximate, and are maintained and monitored in accordance with Covance SOPs. | | |

### Sample Shipment

Ocular tissue samples will be shipped by overnight carrier on dry ice to the following address. Sample shipment will be scheduled following the final collections. Shipments will only be scheduled on a non-holiday Monday, Tuesday, or Wednesday. The Study Monitor and recipient will be notified by e-mail at the time of each shipment. An electronic manifest will be sent at the time of shipment.

Any further analysis of these samples that may be performed has been determined to be outside the scope of this study. Any data generated from these analyses will not be used for interpretation of the results for this study, will not be reported within this study, and will not be used to support any drug safety assessment.

### DATA ANALYSIS

### Statistical Analyses

Statistical analyses may include such parameters as mean and standard deviation, as appropriate.

### DISPOSITION

### Animal Disposition

### Scheduled

Animals will be sacrificed as part of the terminal collection procedure. Carcasses will not be retained.

### Unscheduled

If necessary, at the discretion of the Study Director or laboratory animal veterinarian, animals will be euthanized according to the appropriate method as specified by Covance SOPs.

### Dose Formulation Disposition

Unused dose formulation(s) will be maintained according to Covance SOPs.

### Sample Disposition

All samples will be shipped to another site; no samples will remain at Covance.

### Modified Hackett-Mcdonald Scoring Scale

To be conducted by a veterinary ophthalmologist. Abnormal changes will be recorded according to the following scale.

### Pupillary Light Reflex

Note: Using full illumination with the slit lamp, the following scale is used to score pupillary light reflex.

| Score | Description |
|---|---|
| 0 | Normal pupillary reflex. |
| 1 | Sluggish pupillary reflex. Pupil is relatively dilated with a sluggish pupillary reflex. |
| 2 | Maximally impaired (i.e., fixed) pupillary reflex. Pupil is fully dilated with no pupillary reflex. |
| 3 | Miotic pupil. |

### Conjunctival Congestion (Hyperemia)

Note: The degree of pigmentation in eyes may preclude accurate scoring of this parameter.

| Score | Description |
|---|---|
| 0 | Normal. May appear blanched to reddish pink without perilimbal injection (except at 12:00 and 6:00 positions) with vessels of the palpebral and bulbar conjunctiva easily observed. |
| 1 | A flushed reddish color predominantly confined to the bulbar conjunctiva with some perilimbal injection. Primarily confined to the lower and upper parts of the eye from the 4:00 and 7:00 o'clock and the 11:00 and 1:00 o' clock positions. |
| 2 | Bright red color of the bulbar and palpebral conjunctiva with accompanying perilimbal injection covering at least 75% of the circumference of the perilimbal region. |
| 3 | Dark, beefy red color with congestion of the bulbar and the palpebral conjunctiva along with pronounced perilimbal injection. Petechia may be present on the conjunctiva. The petechiae generally predominate along the nictitating membrane and the upper palpebral conjunctiva. |

### Conjunctival Swelling (Chemosis)

| Score | Description |
|---|---|
| 0 | Normal or no swelling of the conjunctival tissue. |
| 1 | Swelling above normal without eversion of the lids (can be easily ascertained by noting that the upper and lower eyelids are positioned as in |
| | the normal eye); swelling generally starts in the lower cul-de-sac near the inner canthus, which requires slit lamp examination. |
| 2 | Swelling with misalignment of the normal approximation of the lower and upper eyelids; primarily confined to the upper eyelid so that in the initial stages the misapproximation of the eyelids begins by partial eversion of the upper eyelid. In this stage, swelling is confined generally to the upper eyelid, although it exists in the lower cul-de-sac (observed best with the slit lamp). |
| 3 | Swelling definite with partial eversion of the upper and lower eyelids essentially equivalent. This can be easily ascertained by looking at the animal head-on and noticing the positioning of the eyelids; if the eye margins do not meet, eversion has occurred. |
| 4 | Eversion of the upper eyelid is pronounced with less pronounced eversion of the lower eyelid. It is difficult to retract the lids and observe the perilimbal region. |

### Conjunctival Discharge

Note: Discharge is defined as a whitish-gray, serous, purulent, mucoid, and/or bloody material. Normal discharge may include a small amount of clear or mucoid material found in the medial canthus of a substantial number of animal eyes.

| Score | Description |
|---|---|
| 0 | No discharge (except as noted above). |
| 1 | Discharge is above normal and present on the surface of the eye or in the medial canthus, but not on the lids or hairs of the eyelids. |
| 2 | Discharge is abundant, easily observed, and has collected on the lids and around the hairs of the eyelids. |
| 3 | Discharge has been flowing over the eyelids so as to wet the hairs substantially on the skin around the eyes. |

### Cornea

Scores for Corneal Opacity generally require two numbers; the first number indicating the severity of corneal opacity and the second number indicating the estimated area of the involvement. The severity of corneal opacity is graded as follows.

| Score | Description |
|---|---|
| 0 | Normal cornea. Appears with the slit lamp as having a bright grey line on the epithelial surface and a bright grey line on the endothelial surface with a marble-like grey appearance of the stroma. |
| 1 | Some loss of transparency. Only the epithelium and/or the anterior half of the stroma is involved as observed with an optical section of the slit lamp. With diffuse illumination, the underlying structures are clearly visible, although some cloudiness may be readily apparent. |
| 2 | Moderate loss of transparency. The cloudiness extends past the anterior half of the stroma. The affected stroma has lost its marble-like appearance and is homogeneously white. With diffuse illumination, underlying structures are visible, although there may be some loss of detail. |
| 3 | Involvement of the entire thickness of the stroma. With optical section, the endothelial surface is still visible. However, with diffuse illumination, the underlying structures are just barely visible (to the extent that the observer is still able to grade flare, iris vessel congestion, observe for pupillary response, and note lenticular changes). |
| 4 | Involvement of the entire thickness of the stroma. With optical section, the endothelium is not clearly visualized. With diffuse illumination, the underlying structures cannot be seen so that the evaluation of aqueous flare, iris vessel congestion, pupillary response, and lenticular changes is not possible. |

### % Area of Corneal Opacity

| Score | Description |
|---|---|
| 0 | Normal cornea with no area of cloudiness. |
| 1 | 1 to 25% area of stromal cloudiness. |
| 2 | 26 to 50% area of stromal cloudiness. |
| 3 | 51 to 75% area of stromal cloudiness. |
| 4 | 76 to 100% area of stromal cloudiness. |

### Corneal Vascularization

| Score | Description |
|---|---|
| 0 | No corneal vascularization (pannus). |
| 1 | Vascularization is present but vessels have not invaded the entire corneal circumference. Where localized vessel invasion has occurred, they have not penetrated beyond 2 mm. |
| 2 | Vessel invasion is greater than 2 mm in one or more areas, or involves the entire corneal circumference. |

### Aqueous Flare

Note: The intensity of the Tyndall phenomenon (aqueous flare) is scored by comparing the normal Tyndall effect observed when the slit lamp beam passes through the lens with that seen in the anterior chamber. The presence of aqueous flare is presumptive evidence of breakdown of the blood-aqueous barrier.

| Score | Description |
|---|---|
| 0 | No protein is visible in the anterior chamber when viewed by an experienced observer using slit lamp biomicroscopy; a small, bright, focal slit beam of white light; and high magnification. |
| 0.5 | Trace amount of protein is detectable in the anterior chamber. This protein is only visible with careful scrutiny by an experienced observer using slit lamp biomicroscopy; a small, bright, focal slit beam of white light; and high magnification. |
| 1 | Mild amount of protein is detectable in the anterior chamber. The presence of protein in the anterior chamber is immediately apparent to an experienced observer using slit lamp biomicroscopy and high magnification, but such protein is detected only with careful observation with the naked eye and a small, bright, focal slit beam of white light. |
| 2 | Moderate amount of protein is detectable in the anterior chamber. These grades are similar to 1+ but the opacity would be readily visible to the naked eye of an observer using any source of a focused beam of white light. This is a continuum of moderate opacification with 2+ being less apparent than 3+. |
| 3 | Moderate amount of protein is detectable in the anterior chamber. These grades are similar to 1+ but the opacity would be readily visible to the naked eye of an observer using any source of a focused beam of white light. This is a continuum of moderate opacification with 3+ being more apparent than 2+. |
| 4 | Large (severe) amount of protein is detectable in the anterior chamber. Similar to 3+ but the density of the protein approaches that of the lens. Additionally, frank fibrin deposition is frequently seen in acute circumstances. It needs to be noted that because fibrin may persist for a period of time after partial or complete restoration of the blood-aqueous barrier, it is possible to have resorbing fibrin present with lower numeric assignations for flare (e.g., 1+ flare with fibrin). |

### Aqueous Cell

Note: The aqueous or vitreous cell scoring is recorded as two determinations: The first to determine the number of cells visible, the second to describe the coloration of the cells observed (as applicable). The same scoring system used will be used when scoring both aqueous and vitreous cells.

| Score | Description |
|---|---|
| 0 | No cells are seen in a single field of the focused slit lamp beam. No cells are visualized as the slit lamp beam is swept across the anterior chamber. |
| 0.5 | Rare (1-5) cells are seen in a single field of the focused slit lamp beam. When the instrument is held stationary, not every optical section contains circulating cells. |
| 1 | 6-25 cells are seen in a single field of the focused slit lamp beam. When the instrument is held stationary, each optical section of the anterior chamber contains circulating cells. |
| 2 | 26-50 cells are seen in a single field of the focused slit lamp beam. When the instrument is held stationary, each optical section of the anterior chamber contains circulating cells. |
| 3 | 51-100 cells are seen in a single field of the focused slit lamp beam. When the instrument is held stationary, each optical section of the anterior chamber contains circulating cells. Keratic precipitates or cellular deposits on the anterior lens capsule may be present. |
| 4 | Greater than 100 cells are seen in a single field of the focused slit lamp beam. When the instrument is held stationary, each optical section of the anterior chamber contains circulating cells. Keratic precipitates or cellular deposits on the anterior lens capsule may be present. As for fibrin deposition, hypopyon or clumps of cells may persist for some period of time after the active exudation of cells into the anterior chamber has diminished or ceased entirely. Thus, it is possible to have resorbing hypopyon present with lower numeric assignations for cell (e.g., 1+ cell with hypopyon). |

### Aqueous or Vitreous Cell Color

Aqueous or vitreous cell may be observed as white or brown, and will be recorded as one of three categories as follows. Predominantly brown (≥75% brown), predominantly white (≥75% white), or mixed (other ratios of brown and white). Cell color types will not be counted. Rather the ophthalmologist will subjectively categorize the observation.

### Iris Congestion (Hyperemia)

Note: In the following definitions the primary, secondary, and tertiary vessels are utilized as an aid to determining a subjective ocular score for iris congestion. The assumption is made that the greater the hyperemia of the vessels and the more the secondary and tertiary vessels are involved, the greater the intensity of iris involvement. Also, the degree of pigmentation in eyes may preclude accurate scoring of this parameter.

| Score | Description |
|---|---|
| 0 | Normal iris without any hyperemia of the iris vessels. |
| 1 | Minimal injection of secondary vessels but not tertiary. |
| 2 | Minimal injection of the tertiary vessels and minimal to moderate injection of the secondary vessels. |
| 3 | Moderate injection of the secondary and tertiary vessels with a slight swelling of the iris stroma (this gives the iris surface a slightly rugose appearance, which is usually most prominent near the 3:00 and 9:00 positions). |
| 4 | Marked injection of the secondary and tertiary vessels with marked swelling of the iris stroma. The iris appears rugose; may be accompanied by hemorrhage (hyphema) in the anterior chamber. |

### Fluorescein Staining

Note: Fluorescein staining is an indication of corneal epithelial damage. Scores for fluorescein staining are recorded as two scores: the first number indicating the intensity of the staining and the second indicating the estimated area of the involvement.

| Score | Description |
|---|---|
| 0 | Absence of fluorescein staining. |
| 1 | Slight multifocal punctate fluorescein staining. With diffuse illumination the underlying structures are easily visible. (The outline of the pupillary margin is as if there were no fluorescein staining.) |
| 2 | Moderate fluorescein staining confined to a small focus. With diffuse illumination, the underlying structures are clearly visible, although there is some loss of detail. |
| 3 | Marked fluorescein staining. Staining may involve a larger portion of the cornea. With diffuse illumination underlying structures are barely visible but are not completely obliterated. |
| 4 | Extreme fluorescein staining. With diffuse illumination the underlying structures cannot be observed. |

### % Area of Fluorescein Staining

| Score | Description |
|---|---|
| 0 | No area of fluorescein staining. |
| 1 | 1 to 25% area of fluorescein staining. |
| 2 | 26 to 50% area of fluorescein staining. |
| 3 | 51 to 75% area of fluorescein staining. |
| 4 | 76 to 100% area of fluorescein staining. |

Note: The entire area of the cornea that contains stain is scored, regardless of the varying intensities that may be present.

Note: Kikkawa (1972) - reported that 10 to 20% of rabbits examined exhibited focal, punctate fluorescein staining normally. There may be involvement of the whole cornea, or the foci may be limited to one area.

### Lens

The crystalline lens is readily observed with the aid of the slit lamp biomicroscope, and the location of lenticular opacity can readily be discerned by direct and
retro-illumination. The location of lenticular opacities can be arbitrarily divided into the following lenticular regions beginning with the anterior capsule:
Anterior capsule
Anterior subcapsular
Anterior cortical
Equatorial cortical
Nuclear
Posterior cortical
Posterior subcapsular
Posterior capsule

The lens should be evaluated routinely during ocular evaluations and graded as
0 (normal) or the presence of lenticular opacities should be described and the location noted as defined below.

| | |
|---|---|
| Incomplete: | A diffuse lens opacity visible upon gross inspection of the eye with an indirect ophthalmoscope or other focal light source and retroillumination. The view of the fundus is significantly impaired but a red-reflex can still be obtained. Upon slit lamp biomicroscopy the opacity involves multiple regions of the lens. |
| Complete: | A diffuse lens opacity visible upon gross inspection of the eye with an indirect ophthalmoscope or other focal light source. The fundus cannot be seen and a red-reflex cannot be elicited. Upon slit lamp biomicroscopy the entire lens is opaque. |
| Resorbing: | A diffuse lens opacity visible upon gross inspection of the eye with an indirect ophthalmoscope or other focal light source. The fundus may or may not be visible and a red-reflex may or may not be elicited. The lens capsule may be wrinkled and the lens itself is dehydrated and flattened or liquid and soft in appearance. Upon slit lamp biomicroscopy the entire lens is involved in the opacity. |
| Punctate: | A focal or multifocal, discrete, dot-like lens opacity that is visible only to a trained observer with a slit lamp biomicroscope at high magnification. |
| Incipient: | A focal lens opacity that is visible upon gross inspection of the eye with an indirect ophthalmoscope or other focal light source and retroillumination. The view of the fundus is minimally impaired by the opacity. Upon slit lamp biomicroscopy the opacity can be localized to a specific region of the lens and other regions of the lens appear normal. |

### Vitreous Cell

Vitreous cell scores are assigned by using the following estimate of cells per field.

| Score | Description |
|---|---|
| 0 | No cells are seen in a single field of the focused slit lamp beam. |
| 0.5 | Rare (1-5) cells are seen in a single field of the focused slit lamp beam. |
| 1 | 6-25 cells are seen in a single field of the focused slit lamp beam. |
| 2 | 26-50 cells are seen in a single field of the focused slit lamp beam. |
| 3 | 51-100 cells are seen in a single field of the focused slit lamp beam. |
| 4 | Greater than 100 cells are seen in a single field of the focused slit lamp beam. |

### Retina/Fundus

Abnormal findings or an indication of normal (a score of "0") will be recorded as

## Claims

1. A formulation comprising charge-neutral nucleic acid nanoparticles, wherein the nanoparticles each contain a single molecule of nucleic acid which is compacted to its minimal possible size for use in treating an ocular disorder in a mammal, wherein the nanoparticles are ellipsoids, and wherein the formulation is non-surgically administered to the suprachoroidal space (SCS) of an eye of the mammal.

2. The formulation for use according to claim 1, where:
(1) the nanoparticles are ellipsoids with a minor diameter of less than 30 nm;
(2) the nanoparticles are ellipsoids with a minor diameter of less than 20 nm; or
(3) the nanoparticles comprise polyethylene glycol-substituted polylysine.

3. The formulation for use according to claim 1, where the nucleic acid is less than 30 kb or less than 30 kbp.

4. The formulation for use according to claim 1, where:
(1) the mammal has an ocular disorder selected from the group consisting of uveitis, glaucoma, macular edema, diabetic macular edema, retinopathy, age-related macular degeneration, scleritis, optic nerve degeneration, geographic atrophy, choroidal disease, ocular sarcoidosis, optic neuritis, choroidal neovascularization, ocular cancer, retinitis pigmentosa, juvenile onset macular degeneration, a genetic disease, autoimmune diseases affecting the posterior segment of the eye, retinitis and corneal ulcers; or
(2) the mammal has an ocular disorder, selected from the group consisting of choroidal neovascularization, choroidal vascular proliferation, polypoidal choroidal vasculopathy, central sirrus choroidopathy, a multi-focal choroidopathy and choroidal dystrophy.

5. The formulation for use according to claim 1, where the nucleic acid is transcribed to form transcripts and at least one of the transcripts is translated to express a protein.

6. The formulation for use according to claim 1, where the nucleic acid is transcribed to form transcripts and at least one of the transcripts is an anti-sense transcript.

7. The formulation for use according to claim 6, where:
(1) the anti-sense transcript inhibits synthesis of an endogenous protein;
(2) the anti-sense transcript inhibits synthesis of an endogenous protein with a dominant negative mutation; or
(3) the anti-sense transcript inhibits synthesis of an endogenous rhodopsin protein with a dominant negative mutation.

8. The formulation for use according to claim 1, where:
(1) the nucleic acid is translated to express a protein;
(2) the nucleic acid encodes a protein selected from the group consisting of a cytokine, a chemokine, a growth factor, an anti-angiogenesis factor, and an antibody or antibody fragment or construct;
(3) the nucleic acid encodes a protein selected from the group consisting of ABCA4, MYO7A, ND4, GUCY2D, RPE65, Pigment epithelium-derived factor (PEDF), sFlt-1, ABCA; BEST; CORF; CA; CERKL; CHM; CLRN; CNGA; CNGB; CRB; CRX; DHDDS; EYS; FAMA; FSCN; GUCAB; IDHB; IMPDH; IMPG; KLHL; LRAT; MAK; MERTK; NRE; NRL; OFD; PDEA; PDEB; PDEG; PRCD; PROM; PRPF; PRPH; PRPH2; RBP; RDH; RGR; RHO; RLBP; ROM; RP; RPE; RPGR; RS1; SAG; SEMAA; SNRNP; SPATA; TOPORS; TTC; TULP; USHA; ZNF; ABHD12; CDH23; CIB2; CLRN1; DFNB31; GPR98; HARS; MYO7A; PCDH15; USH1C; USH1G; USH2A, ARL6; BBS1; BBS2; BBS4; BBS5; BBS7; BBS9; BBS10; BBS12; CEP290; INPP5E; LZTFL1; MKKS; MKS1; SDCCAG8; TRIM32; TTC8; endostatin, and angiostatin;
(4) the nucleic acid encodes a wild-type form of a protein, where a mutant form of the protein causes retinitis pigmentosa;
(5) the nucleic acid encodes a wild-type form of a protein, where a mutant form of the protein causes a genetic blinding disorder; or
(6) the nucleic acid encodes a wild-type form of a protein, where a mutant form of the protein causes an ocular disease.

9. The formulation for use according to claim 1, where the nucleic acid is DNA or RNA.

10. The formulation for use according to claim 1, where the ocular disease is acquired.

11. The formulation for use according to claim 1, wherein the formulation is administered to the SCS via a hollow microneedle.

## Patentansprüche

1. Formulierung, die ladungsneutrale Nukleinsäure-Nanopartikel umfasst, wobei die Nanopartikel jeweils ein einzelnes Nukleinsäuremolekül enthalten, das zur Verwendung bei der Behandlung einer Augenerkrankung bei einem Säugetier auf seine minimal mögliche Größe komprimiert ist, wobei die Nanopartikel Ellipsoide sind, und wobei die Formulierung nicht-chirurgisch in den suprachoroidalen Raum (SCS) eines Auges des Säugetiers verabreicht wird.

2. Formulierung zur Verwendung nach Anspruch 1, wobei:
(1) die Nanopartikel Ellipsoide mit einem kleinen Durchmesser von weniger als 30 nm sind;
(2) die Nanopartikel Ellipsoide mit einem kleinen Durchmesser von weniger als 20 nm sind; oder
(3) die Nanopartikel Polyethylenglykol-substituiertes Polylysin umfassen.

3. Formulierung zur Verwendung nach Anspruch 1, wobei die Nukleinsäure weniger als 30 kb oder weniger als 30 kbp beträgt.

4. Formulierung zur Verwendung nach Anspruch 1, wobei:
(1) das Säugetier eine Augenerkrankung aufweist, die ausgewählt ist aus der Gruppe bestehend aus Uveitis, Glaukom, Makulaödem, diabetischem Makulaödem, Retinopathie, altersbedingter Makuladegeneration, Skleritis, Sehnervendegeneration, geografischer Atrophie, choroidaler Erkrankung, okularer Sarkoidose, Sehnervenentzündung, choroidaler Neovaskularisation, Augenkrebs, Retinitis pigmentosa, Makuladegeneration jugendlichen Alters, einer genetischen Erkrankung, Autoimmunerkrankungen des hinteren Augenabschnitts, Retinitis und Hornhautgeschwüren; oder
(2) das Säugetier eine Augenerkrankung aufweist, die ausgewählt ist aus der Gruppe bestehend aus choroidaler Neovaskularisation, choroidaler vaskulärer Proliferation, polypoidaler choroidaler Vaskulopathie, zentraler seröser Choroidopathie, einer multifokalen Choroidopathie und choroidaler Dystrophie.

5. Formulierung zur Verwendung nach Anspruch 1, wobei die Nukleinsäure transkribiert wird, um Transkripte zu bilden, und mindestens eines der Transkripte translatiert wird, um ein Protein zu exprimieren.

6. Formulierung zur Verwendung nach Anspruch 1, wobei die Nukleinsäure transkribiert wird, um Transkripte zu bilden, und mindestens eines der Transkripte ein Anti-Sense-Transkript ist.

7. Formulierung zur Verwendung nach Anspruch 6, wobei:
(1) das Anti-Sense-Transkript die Synthese eines endogenen Proteins hemmt;
(2) das Anti-Sense-Transkript die Synthese eines endogenen Proteins mit einer dominant negativen Mutation hemmt; oder
(3) das Anti-Sense-Transkript die Synthese eines endogenen Rhodopsin-Proteins mit einer dominant negativen Mutation hemmt.

8. Formulierung zur Verwendung nach Anspruch 1, wobei:
(1) die Nukleinsäure translatiert wird, um ein Protein zu exprimieren;
(2) die Nukleinsäure für ein Protein kodiert, das ausgewählt ist aus der Gruppe bestehend aus einem Zytokin, einem Chemokin, einem Wachstumsfaktor, einem Anti-Angiogenese-Faktor und einem Antikörper oder Antikörperfragment oder -konstrukt;
(3) die Nukleinsäure für ein Protein kodiert, das ausgewählt ist aus der Gruppe bestehend aus ABCA4, MYO7A, ND4, GUCY2D, RPE65, Pigment-Epithel-Faktor (PEDF), sFlt-1, ABCA; BEST; CORF; CA; CERKL; CHM; CLRN; CNGA; CNGB; CRB; CRX; DHDDS; EYS; FAMA; FSCN; GUCAB; IDHB; IMPDH; IMPG; KLHL; LRAT; MAK; MERTK; NRE; NRL; OFD; PDEA; PDEB; PDEG; PRCD; PROM; PRPF; PRPH; PRPH2; RBP; RDH; RGR; RHO; RLBP; ROM; RP; RPE; RPGR; RS1; SAG; SEMAA; SNRNP; SPATA; TOPORS; TTC; TULP; USHA; ZNF; ABHD12; CDH23; CIB2; CLRN1; DFNB31; GPR98; HARS; MYO7A; PCDH15; USH1C; USH1G; USH2A, ARL6; BBS1; BBS2; BBS4; BBS5; BBS7; BBS9; BBS10; BBS12; CEP290; INPP5E; LZTFL1; MKKS; MKS1; SDCCAG8; TRIM32; TTC8; Endostatin, und Angiostatin;
(4) die Nukleinsäure für eine Wildtyp-Form eines Proteins kodiert, wobei eine mutierte Form des Proteins Retinitis pigmentosa verursacht;
(5) die Nukleinsäure für eine Wildtyp-Form eines Proteins kodiert, wobei eine mutierte Form des Proteins eine genetische Erblindungsstörung verursacht; oder
(6) die Nukleinsäure für eine Wildtyp-Form eines Proteins kodiert, wobei eine mutierte Form des Proteins eine Augenkrankheit verursacht;

9. Formulierung zur Verwendung nach Anspruch 1, wobei die Nukleinsäure DNA oder RNA ist.

10. Formulierung zur Verwendung nach Anspruch 1, wobei die Augenkrankheit erworben ist.

11. Formulierung zur Verwendung nach Anspruch 1, wobei die Formulierung dem SCS über eine hohle Mikronadel verabreicht wird.

## Revendications

1. Formulation comprenant des nanoparticules d'acide nucléique neutres en charge, dans laquelle les nanoparticules contiennent chacune une unique molécule d'acide nucléique qui est compactée à sa taille minimale possible pour être utilisée dans le traitement d'un trouble oculaire chez un mammifère, dans laquelle les nanoparticules sont des ellipsoïdes, et dans laquelle la formulation est administrée de manière non chirurgicale dans l'espace supra-choroïdien (SCS) d'un œil du mammifère.

2. Formulation destinée à être utilisée selon la revendication 1, dans laquelle :
(1) les nanoparticules sont des ellipsoïdes dont le diamètre mineur est inférieur à 30 nm ;
(2) les nanoparticules sont des ellipsoïdes dont le diamètre mineur est inférieur à 20 nm ; ou
(3) les nanoparticules comprennent de la polylysine substituée par du polyéthylène glycol.

3. Formulation destinée à être utilisée selon la revendication 1, dans laquelle l'acide nucléique est inférieur à 30 kb ou inférieur à 30 kbp.

4. Formulation destinée à être utilisée selon la revendication 1, dans laquelle :
(1) le mammifère présente un trouble oculaire sélectionné parmi le groupe consistant en l'uvéite, le glaucome, l'œdème maculaire, l'œdème maculaire diabétique, la rétinopathie, la dégénérescence maculaire liée à l'âge, la sclérite, la dégénérescence du nerf optique, l'atrophie géographique, la maladie de la choroïde, la sarcoïdose oculaire, la névrite optique, la néovascularisation choroïdienne, le cancer oculaire, la rétinite pigmentaire, la dégénérescence maculaire juvénile, une maladie génétique, les maladies auto-immunes affectant le segment postérieur de l'œil, la rétinite et les ulcères cornéens ; ou
(2) le mammifère présente un trouble oculaire sélectionné parmi le groupe consistant en la néovascularisation choroïdienne, la prolifération vasculaire choroïdienne, la vasculopathie polypoïdale choroïdienne, la choriorétinopathie séreuse centrale, une choriorétinopathie multifocale et la dystrophie choroïdienne.

5. Formulation destinée à être utilisée selon la revendication 1, dans laquelle l'acide nucléique est transcrit pour former des transcrits et au moins l'un des transcrits est traduit pour exprimer une protéine.

6. Formulation destinée à être utilisée selon la revendication 1, dans laquelle l'acide nucléique est transcrit pour former des transcrits et au moins l'un des transcrits est un transcrit antisens.

7. Formulation destinée à être utilisée selon la revendication 6, dans laquelle :
(1) le transcrit antisens inhibe la synthèse d'une protéine endogène ;
(2) le transcrit antisens inhibe la synthèse d'une protéine endogène ayant une mutation dominante négative ; ou
(3) le transcrit antisens inhibe la synthèse d'une protéine rhodopsine endogène ayant une mutation dominante négative.

8. Formulation destinée à être utilisée selon la revendication 1, dans laquelle :
(1) l'acide nucléique est traduit pour exprimer une protéine ;
(2) l'acide nucléique code pour une protéine sélectionnée dans le groupe consistant en une cytokine, une chimiokine, un facteur de croissance, un facteur anti-angiogénèse et un anticorps ou un fragment ou une construction d'anticorps ;
(3) l'acide nucléique code pour une protéine sélectionnée dans le groupe consistant en ABCA4, MYO7A, ND4, GUCY2D, RPE65, le facteur dérivé de l'épithélium pigmentaire (PEDF), sFlt-1, ABCA ; BEST ; CORF ; CA ; CERKL ; CHM ; CLRN ; CNGA ; CNGB ; CRB ; CRX ; DHDDS ; EYS ; FAMA ; FSCN ; GUCAB ; IDHB ; IMPDH ; IMPG ; KLHL ; LRAT ; MAK; MERTK; NRE ; NRL; OFD ; PDEA ; PDEB ; PDEG ; PRCD ; PROM ; PRPF ; PRPH ; PRPH2 ; RBP ; RDH ; RGR ; RHO ; RLBP ; ROM ; RP ; RPE ; RPGR ; RS1 ; SAG ; SEMAA ; SNRNP ; SPATA ; TOPORS ; TTC ; TULP ; USHA ; ZNF ; ABHD12 ; CDH23 ; CIB2 ; CLRN1 ; DFNB31 ; GPR98 ; HARS ; MYO7A ; PCDH15 ; USH1C ; USH1G ; USH2A, ARL6 ; BBS1 ; BBS2 ; BBS4 ; BBS5 ; BBS7 ; BBS9 ; BBS10 ; BBS12 ; CEP290 ; INPP5E ; LZTFL1 ; MKKS ; MKS1 ; SDCCAG8 ; TRIM32 ; TTC8 ; endostatine et angiostatine ;
(4) l'acide nucléique code pour une forme sauvage d'une protéine, dans laquelle une forme mutante de la protéine provoque une rétinite pigmentaire ;
(5) l'acide nucléique code pour une forme sauvage d'une protéine, dans laquelle une forme mutante de la protéine provoque une maladie génétique entraînant la cécité ; ou
(6) l'acide nucléique code pour une forme sauvage d'une protéine, dans laquelle une forme mutante de la protéine provoque une maladie oculaire.

9. Formulation destinée à être utilisée selon la revendication 1, dans laquelle l'acide nucléique est l'ADN ou l'ARN.

10. Formulation destinée à être utilisée selon la revendication 1, dans laquelle le trouble oculaire est acquis.

11. Formulation destinée à être utilisée selon la revendication 1, dans lequel la formulation est administrée dans l'espace supra-choroïdien via une micro-aiguille creuse.
